# EUROPEAN PATENT SPECIFICATION

(11) **EP 1 596 834 B1**
(45) Date of publication and mention of the grant of the patent: **30.11.2016**
(21) Application number: 04702174.6
(22) Date of filing: 14.01.2004
(51) Int. Cl.: A61K 9/16, A61K 9/00, A61K 39/00, A61K 31/7088

(54) **MICROPARTICLES COMPRISING POLYNUCLEOTIDES ADSORBED ON THE SURFACE OF OR ENTRAPPED WITHIN THE MICROPARTICLES**
MIKROPARTIKEL, DIE POLYNUKLEOTIDE ENTHALTEN, DIE AN DER PARTIKELOBERFLÄCHE ADSORBIERT ODER ZWISCHEN DEN PARTIKELN EINGESCHLOSSEN SIND
MICROPARTICULES CONTENANT DES POLYNUCLÉOTIDES ADSORBÉS SUR LEUR SURFACE OU INCLUS DANS UNE ESPECE

(30) Priority: 14.01.2003 US 439940 P
(43) Date of publication of application: 23.11.2005
(73) Proprietor: GlaxoSmithKline Biologicals SA, 1330 Rixensart (BE)
(72) Inventor: O'HAGAN, Derek, Berkeley, CA 94705 (US); SINGH, Manmohan, Emeryville, CA 94608 (US)
(74) Representative: Sampson, Catherine
(86) International application number: PCT/US2004/000923
(87) International publication number: WO 2004/065578

(56) References cited:
- WO-A-01/36599
- US-A1- 2002 002 272
- US-A1- 2002 136 776
- US-A1- 2003 138 458
- FATTAL E ET AL: "Biodegradable polyalkylcyanoacrylate nanoparticles for the delivery of oligonucleotides" JOURNAL OF CONTROLLED RELEASE, ELSEVIER SCIENCE PUBLISHERS B.V. AMSTERDAM, NL, vol. 53, no. 1-3, 30 April 1998 (1998-04-30), pages 137-143, XP004121264 ISSN: 0168-3659
- DENIS-MIZE K S ET AL: "PLASMID DNA ADSORBED ONTO CATIONIC MICROPARTICLES MEDIATED TARGET GENE EXPRESSION AND ANTIGEN PRESENTATION BY DENDRITIC CELLS" GENE THERAPY, MACMILLAN PRESS LTD., BASINGSTOKE, GB, vol. 7, no. 24, 2000, pages 2105-2112, XP008009065 ISSN: 0969-7128
- O'HAGAN D T ET AL: "Synergistic adjuvant activity of immunostimulatory DNA and oil/water emulsions for immunization with HIV p55 gag antigen" 10 September 2002 (2002-09-10), VACCINE, BUTTERWORTH SCIENTIFIC. GUILDFORD, GB, PAGE(S) 3389-3398 , XP004378534 ISSN: 0264-410X abstract page 3390, left-hand column, line 16 - page 3395, left-hand column, line 13

## Description

### Statement of Related Application

This application claims the benefit of priority to U.S. provisional patent application number 60/439,940, filed January 14, 2003.

### Field of the Invention

The present invention relates generally to pharmaceutical compositions. In particular, the invention relates to polymer microparticles having adsorbent surfaces wherein biologically active agents, particularly polynucleotide-containing species such as vector constructs (e.g., DNA and RNA vector constructs) or adjuvants (e.g., CpG oligonucleotides) are adsorbed thereto, methods for preparing such microparticles, and uses thereof, including induction of cellular immune responses in vertebrate animals.

### Background

Particulate carriers have been used with adsorbed or entrapped antigens in attempts to elicit adequate immune responses. Such carriers present multiple copies of a selected antigen to the immune system and promote trapping and retention of antigens in local lymph nodes. The particles can be phagocytosed by macrophages and can enhance antigen presentation through cytokine release.

For example, commonly owned International patent application WO 98/33487 and co-pending U.S. Patent Application Serial No. 09/015,652, filed January 29, 1998, describe the use of antigen-adsorbed and antigen-encapsulated microparticles to stimulate immunological responses, including cell-mediated immunological responses, as well as methods of making the microparticles. Polymers used to form the microparticles include poly(lactide) and poly(lactide-co-glycolide), also referred to herein as "PLG".

Commonly owned International patent application WO 00/06123 and co-pending U.S. Patent Application Serial No. 09/715,902 disclose methods of making microparticles having adsorbed macromolecules, including DNA, polypeptides, antigens and adjuvants. The microparticles comprise, for example, a polymer such as a poly(alpha-hydroxy acid) (e.g., PLG), a polyhydroxy butyric acid, a polycaprolactone, a polyorthoester, a polyanhydride, and the like and are formed using, for example, cationic, anionic or nonionic detergents. Microparticles containing anionic detergents, such as PLG microparticles with sodium dodecyl sulfate (SDS), are proposed for the use of positively charged macromolecules, such as polypeptides. Microparticles containing cationic detergents, such as PLG microparticles with CTAB (cetyltrimethylammonium bromide), are proposed for the use of negatively charged macromolecules, such as DNA. The use of such microparticles to stimulate immunological responses, including cell-mediated immunological responses, is also disclosed.

US2002002272, Fattal et al. Journal of Controlled Release, 53, 1-3, 1998, 137-143, WO013699, Denis-Mize et al. Gene Therapy, 7, 24, 2000, 2105-2112, O Hagen. Vaccine.2002.3389-3398, US20031138458 reveal microparticles with polynucleotides adsorbed on their surface. The percentage of loaded DNA is around 1 % w/w with regard to the weight of the microparticles.

At present, there is a desire to increase DNA loading levels from those of the prior art to, *inter alia*, reduce the amount of polymer that is administered to the host animal.

### Summary of the Invention

According to an embodiment of the present invention, microparticles are provided, which comprise: (a) a polymer comprising a poly(α-hydroxy acid), a polyhydroxy butyric acid, a polycaprolactone, a polyorthoester, a polyanhydride, or a polycyanoacrylate; (b) a cationic surfactant; and (c) a first polynucleotide-containing species adsorbed to the microparticles, wherein the first polynucleotide-containing species constitutes 10 to 30 percent.

In several embodiments, the microparticles are formed from a poly(α-hydroxy acid), such as a poly(lactide) ("PLA"), a copolymer of D,L-lactide and glycolide, such as a poly(D,L-lactide-co-glycolide) ("PLG"), or a copolymer of D,L-lactide and caprolactone. Poly(D,L-lactide-co-glycolide) polymers include those having a lactide/glycolide molar ratio ranging, for example, from 20:80 to 80:20, 25:75 to 75:25, 40:60 to 60:40 or 55:45 to 45:55, and having a molecular weight ranging, for example, from 5,000 to 200,00 Daltons, 10,000 to 100,000 Daltons, 20,000 Daltons to 70,000 Daltons, or 40,000 to 50,000 Daltons.

In other aspects of the invention, a microparticle composition is produced, which comprises a pharmaceutically acceptable excipient.

The microparticles may optionally have an additional species, including an additional polynucleotide-containing species, which is: (a) adsorbed to the surface of the microparticles, (b) entrapped within the microparticles, (c) in solution, (d) adsorbed to a separate population of microparticles, and/or (e) entrapped within a separate population of microparticles.

Hence, the invention encompasses a variety of combinations wherein a single polynucleotide-containing species is adsorbed to the microparticles and optionally entrapped within the microparticles. Moreover, the microparticles of the invention may have additional polynucleotide-containing species adsorbed thereon or entrapped therein. Additionally, species other than polynucleotide-containing species, including pharmaceuticals, hormones, enzymes, transcription or translation mediators, metabolic pathway intermediates, immunomodulators, antigens including polypeptide containing antigens, adjuvants including immunological adjuvants, or combinations thereof, may be adsorbed to and/or entrapped within the microparticles. For example, one or more immunological adjuvants may be adsorbed to and/or entrapped within the microparticles.

As further examples, an additional population of microparticles can be provided, (a) having the same polynucleotide-containing species adsorbed thereon, (b) having a different polynucleotide-containing species adsorbed thereon or entrapped therein, (c) having species other than polynucleotide-containing species, for example, one or more immunological adjuvants, adsorbed thereon or entrapped therein. As a specific example, one population of PLG microparticles can be provided having adsorbed thereto a polynucleotide-containing species, while an additional population of PLG microparticles can be provided, which has an immunological adjuvant adsorbed thereon and/or entrapped therein.

The present invention is also directed to immunogenic compositions comprising an immunostimulating amount of a polynucleotide-containing species and an immunostimulating amount of an adjuvant composition, such as those described herein. In some embodiments of the invention, the immunogenic composition comprises a CpG oligonucleotide adjuvant in combination with another polynucleotide-containing species, for example, a vector construct such as an RNA vector construct, a pSINCP vector or a pCMV vector encoding an antigenic polypeptide. Either or both of the polynucleotide-containing species may be adsorbed to the surface of the microparticle.

The polynucleotide-containing species can be, for example, (a) polynucleotide-containing immunological adjuvants, such as CpG oligonucleotides, oligonucleotides having modified backbones, and dsRNA, (b) anti-sense oligonucleotides, and (c) a polynucleotide-containing species that encodes a polypeptide-containing species.

Examples of polynucleotide-containing species that encode a polypeptide-containing species include, for example, (a) a nucleic acid sequence that directly encodes a polypeptide-containing antigen (e.g., an mRNA molecule) or (b) a vector construct that indirectly encodes polypeptide-containing antigen, for example, a vector construct that expresses a heterologous nucleic acid sequence, which in turn encodes a polypeptide-containing antigen (e.g., a DNA vector construct or an RNA vector construct).

Polypeptide-containing antigens can be, for example, tumor antigens or antigen from pathogenic organisms, such as viruses, bacteria, fungi and/or parasites. Thus, in some embodiments, the polypeptide-containing antigen is derived from a virus such as, for example, hepatitis A virus (HAV), hepatitis B virus (HBV), hepatitis C virus (HCV), herpes simplex virus (HSV), human immunodeficiency virus (HIV), cytomegalovirus (CMV), influenza virus (e.g., influenza A virus), and rabies virus. In other embodiments, the polypeptide-containing antigen is derived from a bacterium such as, for example, cholera, diphtheria, tetanus, streptococcus (e.g., streptococcus A and B), pertussis, *Neisseria meningitidis* (e.g., meningitis A, B, C, W, Y), *Neisseria gonorrhoeae*, *Helicobacter pylori*, *Haemophilus influenza* (e.g., *Haemophilus influenza* type B) and anthrax. In still other embodiments, the polypeptide-containing antigen is derived from a parasite such as, for example, a malaria parasite.

In still other embodiments, the invention is directed to methods of delivering polynucleotide-containing species to a host animal, which comprises administering to the host animal any of the microparticle compositions described above. The host animal is preferably a vertebrate animal, more preferably a mammal, and even more preferably a human.

The present invention is also directed to methods of stimulating an immune response in a host animal, which comprises administering to the animal any of the microparticle compositions described above in an amount effective to induce an immune response. The immune response can be a cellular and/or a humoral immune response.

The present invention is directed to methods of stimulating a Th1 immune response, or a CTL response, or lyphoproliferation, or cytokine production in a host animal comprising administering to the animal any of the immunogenic microparticle compositions described herein in an amount effective to induce the Th1 immune response, or the CTL response, or the lyphoproliferation, or the cytokine production.

In other embodiments, the invention is directed to the use for the manufacture of a medicament for immunization, which comprise administering to a host animal a therapeutically effective amount of any of the microparticle compositions described above.

The present invention is also directed to the use for the manufacture of a medicament for immunizing a host animal against, e.g., a tumor or a viral, bacterial, or parasitic infection comprising administering to the animal an immunogenic microparticle composition described herein in an amount effective to induce a protective response.

Delivery of the microparticle compositions of the invention may be performed by any known method, including direct injection (e.g., subcutaneously, intraperitoneally, intravenously or intramuscularly).

Hence, according to some embodiments of the present invention, compositions and methods are provided which treat, including prophylactically and/or therapeutically immunize, a host animal, e.g., against viral, fungal, mycoplasma, bacterial, or protozoan infections, as well as to tumors. The uses of the present invention for the manufacture of a medicament are directed to conferring prophylactic and/or therapeutic immunity to a mammal, preferably a human. The uses of the present invention can also be practiced on animals, other than humans, including biomedical research applications.

Other embodiments of the present invention are directed to methods for producing the above microparticles. For example, the above microparticles can be produced by a method that comprises: (a) forming a w/o/w emulsion comprising the polymer and the cationic surfactant; (b) removing the organic solvent from the emulsion, to form the microparticles; and (c) adsorbing the polynucleotide-containing species to the microparticles.

One particular advantage of the microparticles with adsorbed polynucleotide-containing species of the present invention is the ability to generate immune responses in a vertebrate subject. In addition to a conventional antibody response, the compositions herein described can provide for, e.g., the association of the expressed antigens with class I MHC molecules such that an *in vivo* cellular immune response to the antigen of interest can be mounted which stimulates the production of CTLs to allow for future recognition of the antigen. Furthermore, an antigen-specific response by helper T-cells may be elicited. Accordingly, the uses of the present invention for the manufacture of a medicament are directed to eliciting cellular and/or humoral immune responses to a variety of antigens. As a specific example, antigens derived from viral pathogens can induce antibodies, T-cell helper epitopes and T-cell cytotoxic epitopes. Such antigens include those encoded by human and animal viruses and can correspond to either structural or non-structural proteins.

### Detailed Description of the Invention

The practice of the present invention will employ, unless otherwise indicated, conventional methods of chemistry, polymer chemistry, biochemistry, molecular biology, immunology and pharmacology, within the skill of the art. Such techniques are explained fully in the literature. See, e.g., Remington's Pharmaceutical Sciences, 18th Edition (Easton, Pennsylvania: Mack Publishing Company, 1990); Methods In Enzymology (S. Colowick and N. Kaplan, eds., Academic Press, Inc.); Handbook of Experimental Immunology, Vols. I-IV (D.M. Weir and C.C. Blackwell, eds., 1986, Blackwell Scientific Publications); Sambrook, et al., Molecular Cloning: A Laboratory Manual (2nd Edition, 1989); Handbook of Surface and Colloidal Chemistry (Birdi, K.S., ed, CRC Press, 1997) and Seymour/Carraher's Polymer Chemistry (4th edition, Marcel Dekker Inc., 1996).

As used in this specification and any appended claims, the singular forms "a," "an" and "the" include plural references unless the content clearly dictates otherwise. Thus, for example, the term "microparticle" refers to one or more microparticles, and the like.

Unless stated otherwise, all percentages and ratios herein are given on a weight basis.

### A. Definitions

In describing the present invention, the following terms will be employed, and are intended to be defined as indicated below.

The term "microparticle" as used herein, refers to a particle of about 10 nm to about 150 µm in diameter, more typically about 200 nm to about 30 µm in diameter, and even more typically about 500 nm to about 10-20 µm in diameter. The microparticles of the present invention may aggregate into larger masses under some circumstances. As a specific example, the microparticles of the present invention having adsorbed DNA may be, for instance, about 0.5-2 µm in diameter pre-lyophilization, while the same particles may be, for instance, in aggregates having a diameter of about 5-15 µm post-lyophilization. The microparticle will generally be of a diameter that permits parenteral or mucosal administration without occluding needles and capillaries. Microparticle size is readily determined by techniques well known in the art, such as photon correlation spectroscopy, laser diffractometry and/or scanning electron microscopy. The term "particle" may also be used to denote a microparticle as defined herein.

Polymer microparticles for use herein are typically formed from materials that are sterilizable, substantially non-toxic and biodegradable. Such materials include biodegradable polymers such as poly(α-hydroxy acid), polyhydroxybutyric acid, polycaprolactone, polyorthoester, polyanhydride, and polycyanoacrylate (e.g., polyalkylcyanoacrylate or "PACA"). More typically, microparticles for use with the present invention are polymer microparticles derived from a poly(α-hydroxy acids), in particular, from a poly(lactide) ("PLA") or a copolymer of D,L-lactide and glycolide, such as a poly(D,L-lactide-co-glycolide) ("PLG"), or a copolymer of D,L-lactide and caprolactone. The polymer microparticles may be derived from any of various polymeric starting materials which have a variety of molecular weights and, in the case of the copolymers such as PLG, a variety of monomer (e.g., lactide:glycolide) ratios, the selection of which will be largely a matter of choice, depending in part on the coadministered species. These parameters are discussed more fully below.

The term "surfactant" as used herein includes detergents, dispersing agents, suspending agents, and emulsion stabilizers. Cationic surfactants for use in the microparticle compositions of the present invention include, but are not limited to, cetyltrimethylammonium bromide or "CTAB" (e.g., cetrimide), benzalkonium chloride, DDA (dimethyl dioctodecyl ammonium bromide), DOTAP (dioleoyl-3-trimethylammonium-propane), and the like. Anionic surfactants include, but are not limited to, SDS (sodium dodecyl sulfate), SLS (sodium lauryl sulfate), DSS (disulfosuccinate), sulphated fatty alcohols, and the like. Nonionic surfactants include, but are not limited to, PVA, povidone (also known as polyvinylpyrrolidone or PVP), sorbitan esters, polysorbates, polyoxyethylated glycol monoethers, polyoxyethylated alkyl phenols, poloxamers, and the like.

The term "macromolecule" as used herein refers to, without limitation, a pharmaceutical, a polynucleotide, a polypeptide, a hormone, an enzyme, a transcription or translation mediator, an intermediate in a metabolic pathway, an immunomodulator, an antigen, an adjuvant, or combinations thereof. Particular macromolecules for use with the present invention are described in more detail below.

The term "pharmaceutical" refers to biologically active compounds such as antibiotics, antiviral agents, growth factors, hormones, and the like.

The term "adjuvant" refers to any substance that assists or modifies the action of a pharmaceutical, including but not limited to immunological adjuvants, which increase or diversify the immune response to an antigen. Hence, immunological adjuvants are compounds which are capable of potentiating an immune response to antigens. Immunological adjuvants can potentiate both humoral and cellular immunity.

A "polynucleotide" is a nucleic acid polymer. In some instances, for example, CpG oligonucleotides, the polynucleotide acts as an adjuvant. In other instances, for example, vector constructs, the polynucleotide encodes one or more biologically active (e.g., immunogenic or therapeutic) proteins or polypeptides. A polynucleotide can include as little as 5, 6, 7 or 8 nucleotides, for instance, in the case where the polynucleotide is a CpG oligonucleotide. (CpG oligonucleotides vary widely in size, including, for example, 5, 10, 20, 50, 100, 200 or 500 nucleotides, and so forth, with 20-40 nucleotides being typical.) Furthermore, a "polynucleotide" can include both double- and single-stranded sequences and refers to, but is not limited to, cDNA from viral, procaryotic or eucaryotic mRNA, genomic RNA and DNA sequences from viral (e.g. RNA and DNA viruses and retroviruses) or procaryotic DNA, and synthetic DNA sequences. The term also captures sequences that include any of the known base analogs of DNA and RNA. The term further includes modifications, such as deletions, additions and substitutions (generally conservative in nature), to a native sequence, for example, where the nucleic acid molecule encodes a therapeutic or antigenic protein. These modifications may be deliberate, as through site-directed mutagenesis, or may be accidental, such as through mutations of hosts that produce antigens.

As used herein, the phrase "nucleic acid" refers to DNA, RNA, or chimeras formed therefrom.

A "polynucleotide-containing species" is a molecule, at least a portion of which is a polynucleotide. Examples include CpG nucleotides, RNA vector constructs, DNA vector constructs and so forth.

The terms "polypeptide" and "protein" refer to a polymer of amino acid residues and are not limited to a minimum length of the product. Thus, peptides, oligopeptides, dimers, multimers, and the like, are included within the definition. Both full-length proteins and fragments thereof are encompassed by the definition. The terms also include modifications, such as deletions, additions and substitutions (generally conservative in nature), to a native sequence, for example, such that the protein maintains the ability to elicit an immunological response or have a therapeutic effect on a subject to which the protein is administered.

A "polypeptide-containing species" is a molecule, at least a portion of which is a polypeptide. Examples include polypeptides, proteins including glycoproteins, saccharide antigens conjugated to carrier proteins, and so forth.

By "antigen" is meant a molecule that contains one or more epitopes capable of stimulating a host's immune system to make a cellular antigen-specific immune response when the antigen is presented, or a humoral antibody response. An antigen may be capable of eliciting a cellular or humoral response by itself or when present in combination with another molecule.

An "epitope" is that portion of an antigenic molecule or antigenic complex that determines its immunological specificity. An epitope is within the scope of the present definition of antigen. Commonly, an epitope is a polypeptide or polysaccharide in a naturally occurring antigen. In artificial antigens it can be a low molecular weight substance such as an arsanilic acid derivative. An epitope will react specifically *in vivo* or *in vitro* with, for example, homologous antibodies or T lymphocytes. Alternative descriptors are antigenic determinant, antigenic structural grouping and haptenic grouping.

Typically, a linear epitope will include between about 5-15 amino acids. Epitopes of a given protein can be identified using any number of epitope mapping techniques, well known in the art. See, e.g., Epitope Mapping Protocols in Methods in Molecular Biology, Vol. 66 (Glenn E. Morris, Ed., 1996) Humana Press, Totowa, New Jersey. For example, linear epitopes may be determined by, for example, concurrently synthesizing large numbers of peptides on solid supports, the peptides corresponding to portions of the protein molecule, and reacting the peptides with antibodies while the peptides are still attached to the supports. Such techniques are known in the art and described in, e.g., U.S. Patent No. 4,708,871; Geysen et al. (1984) Proc. Natl. Acad. Sci. USA 81:3998-4002; Geysen et al. (1986) Molec. Immunol. 23:709-715. Similarly, conformational epitopes are readily identified by determining spatial conformation of amino acids such as by, e.g., x-ray crystallography and 2-dimensional nuclear magnetic resonance. See, e.g., *Epitope Mapping Protocols*, *supra.*

The term "antigen" as used herein denotes both subunit antigens, i.e., antigens which are separate and discrete from a whole organism or tumor cell with which the antigen is associated in nature, as well as killed, attenuated or inactivated bacteria, viruses, parasites, fungi or other pathogens or tumor cells. Antibodies such as anti-idiotype antibodies, or fragments thereof, and synthetic peptide mimotopes, which can mimic an antigen or antigenic determinant, are also captured under the definition of antigen as used herein.

Similarly, an oligonucleotide or polynucleotide that expresses an immunogenic protein, or antigenic determinant *in vivo*, such as in nucleic acid immunization applications, is also included in the definition of antigen herein.

Furthermore, for purposes of the present invention, an "antigen" refers to a protein which includes modifications, such as deletions, additions and substitutions (generally conservative in nature), to the native sequence, so long as the protein maintains the ability to elicit an immunological response. These modifications may be deliberate, as through site-directed mutagenesis, or may be accidental, such as through mutations of hosts which produce the antigens.

An "immunological response" to an antigen or composition is the development in a subject of a humoral and/or a cellular immune response to molecules present in the composition of interest. For purposes of the present invention, a "humoral immune response" refers to an immune response mediated by antibody molecules, while a "cellular immune response" is one mediated by T-lymphocytes and/or other white blood cells. One important aspect of cellular immunity involves an antigen-specific response by cytolytic T-cells ("CTLs"). CTLs have specificity for peptide antigens that are presented in association with proteins encoded by the major histocompatibility complex (MHC) and expressed on the surfaces of cells. CTLs help induce and promote the intracellular destruction of intracellular microbes, or the lysis of cells infected with such microbes. Another aspect of cellular immunity involves an antigen-specific response by helper T-cells. Helper T-cells act to help stimulate the function, and focus the activity of, nonspecific effector cells against cells displaying peptide antigens in association with MHC molecules on their surface. A "cellular immune response" also refers to the production of cytokines, chemokines and other such molecules produced by activated T-cells and/or other white blood cells, including those derived from CD4+ and CD8+ T-cells.

A composition such as an immunogenic composition or vaccine that elicits a cellular immune response may serve to sensitize a vertebrate subject by the presentation of antigen in association with MHC molecules at the cell surface. The cell-mediated immune response is directed at, or near, cells presenting antigen at their surface. In addition, antigen-specific T-lymphocytes can be generated to allow for the future protection of an immunized host.

The ability of a particular antigen or composition to stimulate a cell-mediated immunological response may be determined by a number of assays, such as by lymphoproliferation (lymphocyte activation) assays, CTL cytotoxic cell assays, by assaying for T-lymphocytes specific for the antigen in a sensitized subject, or by measurement of cytokine production by T cells in response to restimulation with antigen. Such assays are well known in the art. See, e.g., Erickson et al., J. Immunol. (1993) 151:4189-4199; Doe et al., Eur. J. Immunol. (1994) 24:2369-2376; and the examples below.

The antigen of interest may also elicit an antibody-mediated immune response. Hence, an immunological response may include one or more of the following effects: the production of antibodies by B-cells; and/or the activation of suppressor T-cells and/or γδ T-cells directed specifically to an antigen or antigens present in the composition or vaccine of interest. These responses may serve to neutralize infectivity, and/or mediate antibody-complement, or antibody dependent cell cytotoxicity (ADCC) to provide protection to an immunized host. Such responses can be determined using standard immunoassays and neutralization assays, well known in the art, for instance, radioimmunoassays and ELISAs.

A composition which contains a selected antigen adsorbed to a microparticle, displays "enhanced immunogenicity" when it possesses a greater capacity to elicit an immune response than the immune response elicited by an equivalent amount of the antigen when delivered without association with the microparticle. Thus, a composition may display "enhanced immunogenicity," for example, because the antigen is more strongly immunogenic by virtue of adsorption to the microparticle, or because a lower dose of antigen is necessary to achieve an immune response in the subject to which it is administered. Such enhanced immunogenicity can be determined, for example, by administering the microparticle/antigen composition, and antigen controls, to animals and comparing assay results of the two.

As used herein, "treatment" (including variations thereof, for example, "treat" or "treated") refers to any of (i) the prevention of a pathogen or disorder in question (e.g. cancer or infection by a pathogen, as in a traditional vaccine), (ii) the reduction or elimination of symptoms, and (iii) the substantial or complete elimination of the pathogen or disorder in question. Treatment may be effected prophylactically (prior to the pathogen or disorder in question) or therapeutically (following arrival of the same).

The terms "effective amount" or "pharmaceutically effective amount" of a composition comprising the microparticles of the present invention refer herein to a sufficient amount of the microparticle composition to treat or diagnose a condition of interest. The exact amount required will vary from subject to subject, depending, for example, on the species, age, and general condition of the subject; the severity of the condition being treated; the particular adsorbed/entrapped species of interest; in the case of an immunological response, the capacity of the subject's immune system to synthesize antibodies and the degree of protection desired; and its mode of administration, among other factors. An appropriate "effective" amount in any individual case may be determined by one of ordinary skill in the art. Thus, a "therapeutically effective amount" will typically fall in a relatively broad range that can be determined through routine trials.

By "vertebrate subject" is meant any member of the subphylum cordata, including, without limitation, mammals such as cattle, sheep, pigs, goats, horses, and humans; domestic animals such as dogs and cats; and birds, including domestic, wild and game birds such as cocks and hens including chickens, turkeys and other gallinaceous birds. The term does not denote a particular age. Thus, both adult and newborn animals are covered.

By "pharmaceutically acceptable" or "pharmacologically acceptable" is meant a material which is not biologically or otherwise undesirable, i.e., the material may be administered to an individual along with the microparticle formulation without causing any excessively undesirable biological effects in the individual or interacting in an excessively deleterious manner with any of the components of the composition in which it is contained.

The term "excipient" refers to essentially any accessory substance which may be present in the finished dosage form. For example, the term "excipient" includes vehicles, binders, disintegrants, fillers (diluents), lubricants, glidants (flow enhancers), compression aids, colors, sweeteners, preservatives, suspending/dispersing agents, film formers/coatings, flavors and printing inks.

By "physiological pH" or a "pH in the physiological range" is meant a pH in the range of approximately 7.2 to 8.0 inclusive, more typically in the range of approximately 7.2 to 7.6 inclusive.

As used herein, the phrase "oligonucleotide comprising at least one CpG motif' refers to a polynucleotide comprising at least one CpG dinucleotide. Oligonucleotides comprising at least one CpG motif can comprise multiple CpG motifs. These oligonucleotides are also known as "CpG oligonucleotides" in the art. As used herein, the phrase "CpG motif' refers to a dinucleotide portion(s) of an oligonucleotide, which comprises a cytosine nucleotide followed by a guanosine nucleotide. 5-methylcytosine can also be used in place of cytosine.

Oligonucleotides comprising CpG motifs mixed with antigens have been demonstrated to induce strong Th1 immune responses. Roman et al., Nat. Med., 1997, 3, 849-854; Weiner et al., Proc. Natl. Acad. Sci. USA, 1997, 94, 10833-10837; Davis et al., J. Immunol., 1998, 160, 870-876; Chu et al., J. Exp. Med., 1997, 186, 1623-1631; Lipford et al., Eur. J. Immunol., 1997, 27, 2340-2344; and Moldoveanu et al., Vaccine, 1988, 16, 1216-1224. Unmethylated CpG dinucleotides are relatively common in bacterial DNA, but are underrepresented and methylated in vertebrate DNA. Bird, Trends Genet., 1987, 3, 342-347. Bacterial DNA or synthetic oligonucleotides containing unmethylated CpG motifs are also known to induce immune responses including, for example, B cell proliferation, interleukin-6 and immunoglobulin secretion, and apoptosis resistance. Krieg et al., Nature, 1995, 374, 546-549; Klinman et al., Proc. Natl. Acad. Sci. USA, 1996, 93, 2879-2883; Ballas et al., J. Immunol., 1996, 157, 1840-1845; Cowdery et al., J. Immunol., 1996, 156, 4570-4575; Halpern et al., Cell. Immunol., 1996, 167, 72-78; Yamamoto et al., Jpn. J. Cancer Res., 1988, 79, 866-873; Stacey et al., J. Immunol., 1996, 157, 2116-2122; Messina et al., J. Immunol., 1991, 147, 1759-1764; Yi et al., J. Immunol., 1996, 157, 4918-4925; Yi et al., J. Immunol., 1996, 157, 5394-5402; Yi et al., J. Immunol., 1998, 160, 4755-4761; and Yi et al., J. Immunol., 1998, 160, 5898-5906; PCT Publication WO 96/02555; PCT Publication WO 98/16247; PCT Publication WO 98/18810; PCT Publication WO 98/40100; PCT Publication WO 98/55495; PCT Publication WO 98/37919; and PCT Publication WO 98/52581.

CpG oligonucleotides can be prepared using conventional oligonucleotide synthesis techniques well known to the skilled artisan. CpG oligonucleotides can comprise a modified backbone, such as a phosphorothioate or peptide nucleic acid, so as to confer nuclease resistance to the oligonucleotide. Modified backbones are well known to those skilled in the art. Preferred peptide nucleic acids are described in detail in U.S. Patent Numbers 5,821,060, 5,789,573, 5,736,392, and 5,721,102, Japanese Patent No. 10231290, European Patent No. 839,828, and PCT Publication Numbers WO 98/42735, WO 98/42876, WO 98/36098, WO 98/27105, WO 98/20162, WO 98/16550, WO 98/15648, WO 98/04571, WO 97/41150, WO 97/39024, and WO 97/38013, the disclosures of which are incorporated herein by reference in their entirety.

CpG oligonucleotides typically comprise between about 6 and about 100 nucleotides, more typically between about 8 and about 50 nucleotides, most typically between about 10 and about 40 nucleotides. In addition, the CpG oligonucleotides of the invention can comprise substitutions of the sugar moieties and nitrogenous base moieties. Preferred CpG oligonucleotides are disclosed in, for example, Krieg et al., Proc. Natl. Acad. Sci. USA, 1998, 95, 12631-12636, Klinman et al., Proc. Natl. Acad. Sci. USA, 1996, 93, 2879-2883, Weiner et al., Proc. Natl. Acad. Sci. USA, 1997, 94, 10833-10837, Chu et al., J. Exp. Med., 1997, 186, 1623-1631, Brazolot-Millan et al., Proc. Natl. Acad. Sci. USA, 1998, 95, 15553-15558, Ballas et al., J. Immunol., 1996, 157, 1840-1845, Cowdery et al., J. Immunol., 1996, 156, 4570-4575, Halpern et al., Cell. Immunol., 1996, 167, 72-78, Yamamoto et al., Jpn. J. Cancer Res., 1988, 79, 866-873, Stacey et al., J. Immunol., 1996, 157, 2116-2122, Messina et al., J. Immunol., 1991, 147, 1759-1764, Yi et al., J. Immunol., 1996, 157, 4918-4925, Yi et al., J. Immunol., 1996, 157, 5394-5402, Yi et al., J. Immunol., 1998, 160, 4755-4761, Roman et al., Nat. Med., 1997, 3, 849-854, Davis et al., J. Immunol., 1998, 160, 870-876, Lipford et al., Eur. J. Immunol., 1997, 27, 2340-2344, Moldoveanu et al., Vaccine, 1988, 16, 1216-1224, Yi et al., J. Immunol., 1998, 160, 5898-5906, PCT Publication WO 96/02555, PCT Publication WO 98/16247, PCT Publication WO 98/18810, PCT Publication WO 98/40100, PCT Publication WO 98/55495, PCT Publication WO 98/37919, and PCT Publication WO 98/52581, and commonly owned WO 02/26209.

As used herein, "dsRNA" refers to double-stranded RNA, which can be obtained from various sources. A number of organisms naturally produce dsRNA, including yeasts and viruses. dsRNA from such sources is generally made up of intermittent riboguanylic acid-ribocytidylic acid ([rG-rC]) and riboadenylic acid-polribouridylic acid ([rA-rU]) base pairs. It is believed that all viruses except single-stranded DNA viruses, produce dsRNA. Viral dsRNA generally exists either in the form of duplexes of complementary RNA strands or in the form of intramolecular secondary structure within single-stranded RNA. Viral sources of dsRNA for dsRNA viruses (genomic), ssRNA viruses (transcription intermediates), dsDNA viruses (symmetrical transcription followed by RNA-RNA annealing), and retroviruses (secondary structure in viral mRNA) are known and described in, e.g., Majde, J.A., J. Intenfer. Cytokine Res. (2000) 20:259-272 and Jacobs and Langland, Virology (1996) 219:339-349. Particular sources of viral dsRNA include, but are not limited to, dsRNAs from Mengo virus-infected cells (Falcoff et al., Antimicrob. Agents Chemother. (1973) 3:590-598); dsRNAs from reoviruses and fungal viruses (Field et al., Proc. Natl. Acad. Sci. USA (1967) 58:1004-1010, De Benedetti et al., J. Virol. (1985) 54:408-413); retrovirus dsRNA (Jacobs and Langland, Virology (1996) 219:339-349), such as from HIV-1 (Maitra et al., Virology (1994) 204:823-827); dsRNA extracted from picornavirus-infected cells (Falcoff et al., Antimicrob. Agents Chemother. (1973) 3:590-598); dsRNA from influenza-infected lungs (Majde et al., Microb. Pathogen. (1991) 10:105-115); dsRNA from infected plant cells (Lin and Langenberg, Virology (1985) 142:291-298); dsRNA from togaviruses (Stollar, B.D., Crit. Rev. Biochem. (1975) 3:45-69); dsRNA from rubella-virus infected cells (Lee et al., Virology (1994) 200:307-312); dsRNA from Semliki Forest virus-infected cells (Lee et al., Virology (1994) 200:307-312); dsRNA from dengue virus-infected cells (MacKenzie et al., Virology (1996) 220:232-240); the dsRNAs known as Larifan (Riga, Latvia) and Ridostin ("Diapharam" NOP "VECTOR," Berdsk, Russia). Any of these various dsRNAs, as well as dsRNAs from other sources, will find use with the present compositions and methods.

DsRNA from infected cells is readily obtained using standard methods of nucleic acid extraction, such as phenol extraction techniques, and as described in several of the publications above. See, e.g., Falcoff et al., Antimicrob. Agents Chemother. (1973) 3:590-598; Fayet et al., Prog. Immunobiol. Standard. (1972) 5:267-273; Majde et al., Microb. Pathogen. (1991) 10:105-115).

A number of synthetic dsRNAs are also known and will find use herein and are synthesized using techniques well known and described in the art. Such synthetic dsRNAs include, but are not limited to, polyriboinosinic-polyribocytidylic acid (poly[rI-rC]) and polyriboguanylic-polyribocytidylic acid (poly[rG-rC]) (see, e.g., Michelson et al., Prog. Nuc. Acid Res. Mol. Biol. (1967) 6:83-141); polyriboadenylic-polyribouridylic acid (poly[rA-rU]); low molecular weight dsRNA of mixed base composition, such as, but not limited to, a synthetic dsRNA with 309 bp (Haines et al., J. Biol. Chem. (1992) 267:18315-18319); as well as the synthetic mismatched dsRNAs described in, e.g., U.S. Patent Nos. 5,906,980 and 5,258,369. Moreover, dsRNAs with modified backbones can be made using techniques well known in the art. Further information can be found, for example, in commonly owned PCT/US02/30423.

As used herein, the phrase "vector construct" generally refers to any assembly that is capable of directing the expression of a nucleic acid sequence(s) or gene(s) of interest. A vector construct typically includes transcriptional promoter/enhancer or locus defining element(s), or other elements which control gene expression by other means such as alternate splicing, nuclear RNA export, post-translational modification of messenger, or post-transcriptional modification of protein. In addition, the vector construct typically includes a sequence which, when transcribed, is operably linked to the sequence(s) or gene(s) of interest and acts as a translation initiation sequence. The vector construct may also optionally include a signal that directs polyadenylation, a selectable marker, as well as one or more restriction sites and a translation termination sequence. In addition, if the vector construct is placed into a retrovirus, the vector construct may include a packaging signal, long terminal repeats (LTRs), and positive and negative strand primer binding sites appropriate to the retrovirus used (if these are not already present).

As used herein, a "DNA vector construct" refers to a DNA molecule that is capable of directing its own amplification or self-replication in vivo, typically within a target cell.

One specific type of DNA vector construct is a plasmid, which is a circular episomal DNA molecule capable of autonomous replication within a host cell. Typically, a plasmid is a circular double stranded DNA, loop into which additional DNA segments can be ligated. pCMV is one specific plasmid that is well known in the art. A preferred pCMV vector is one which contains the immediate-early enhancer/promoter of CMV and a bovine growth hormone terminator. It is described in detail in Chapman, B. S., et al. 1991. "Effect of intron A from human cytomegalovirus (Towne) immediate-early gene on heterologous expression in mammalian cells." Nucleic Acids Res. 19:3979-86.

Other DNA vector constructs are known, which are based on RNA viruses. These DNA vector constructs typically comprise a promoter that functions in a eukaryotic cell, 5' of a cDNA sequence for which the transcription product is an RNA vector construct (e.g., an alphavirus RNA vector replicon), and a 3' termination region. The RNA vector construct preferably comprises an RNA genome from a picornavirus, togavirus, flavivirus, coronavirus, paramyxovirus, yellow fever virus, or alphavirus (e.g., Sindbis virus, Semliki Forest virus, Venezuelan equine encephalitis virus, or Ross River virus), which has been modified by the replacement of one or more structural protein genes with a selected heterologous nucleic acid sequence encoding a product of interest. The RNA vector constructs can be obtained by transcription *in vitro* from a DNA template. Specific examples include Sindbis-virus-based plasmids (pSIN) such as pSINCP, described, for example, in U.S. Patents 5,814,482 and 6,015,686, as well as in International Patent Applications WO 97/38087, WO 99/18226 and commonly owned WO 02/26209. The construction of such vectors, in general, is described in U.S. Patents 5,814,482 and 6,015,686. Briefly, RNA is obtained from an RNA virus, then cDNA is synthesized by PCR amplification using appropriate primers for particular genes or portions of the RNA virus, which primers may also contain additional restriction sites as necessary. The cDNA fragments are then cloned into a plasmid and transformed into an appropriate host such as *E. coli.* Positive colonies are grown for plasmid purification, and then plasmids are assembled into the desired vector with a portion having heterologous DNA such as a desired gene coding for an antigen.

Other examples of vector constructs include RNA vector constructs (e.g., alphavirus vector constructs) and the like.

As used herein, "RNA vector construct", "RNA vector replicon" and "replicon" refer to an RNA molecule that is capable of directing its own amplification or self-replication in vivo, typically within a target cell. The RNA vector construct is used directly, without the requirement for introduction of DNA into a cell and transport to the nucleus where transcription would occur. By using the RNA vector for direct delivery into the cytoplasm of the host cell, autonomous replication and translation of the heterologous nucleic acid sequence occurs efficiently.

In some embodiments, the RNA vector construct is obtained by in vitro transcription from a DNA-based vector construct. For example, the RNA vector construct may be derived from the genome of an alphavirus, more preferably from Sindbis virus (SIN), Semliki Forest virus (SFV), Venezuelan equine encephalitis virus (VEE), or Ross River virus (RRV). Or the RNA vector construct may be derived from a virus other than an alphavirus. Such other viruses used for the derivation of RNA vector constructs include positive-stranded RNA viruses, for example, picornaviruses, flaviviruses, rubiviruses, or coronaviruses. Compositions and methods for in vitro transcription of alphavirus-based RNA vectors is provided in detail elsewhere (see U.S. Patent 5,842,723, commonly owned WO 02/26209, and Polo et al., 1999, PNAS 96:4598-603).

An alphavirus-derived RNA vector replicon typically contains the following elements: 5' viral sequences required in cis for replication (also referred to as 5' CSE), sequences which, when expressed, code for biologically active alphavirus nonstructural proteins (e.g., nsP1, nsP2, nsP3, nsP4), 3' viral sequences required in cis for replication (also referred to as 3' CSE), and a polyadenylate tract. An alphavirus-derived RNA vector replicon also may contain a viral subgenomic "junction region" promoter, sequences from one or more structural protein genes or portions thereof, extraneous nucleic acid molecule(s) that are of a size sufficient to allow production of viable virus, as well as heterologous sequence(s) to be expressed.

### B. General Methods

As noted above, various embodiments of the present invention are directed to microparticles that comprise: (a) a biodegradable polymer, for example, one comprising a poly(α-hydroxy acid), a polyhydroxy butyric acid, a polycaprolactone, a polyorthoester, a polyanhydride, or a polycyanoacrylate; (b) a cationic surfactant; and (c) a first polynucleotide-containing species adsorbed to the microparticles, wherein the first polynucleotide-containing species constitutes at least 5 percent of the total weight of the microparticles, more typically 10 to 30 percent, and even more typically 10 to 20 percent.

The present inventors have unexpectedly found that polynucleotide-containing species can be adsorbed to microparticles at high levels. For example, microparticles containing 8, 12, 16 and 20 wt% of an adsorbed polynucleotide-containing species (i.e., a polypeptide-antigen-encoding vector construct, more specifically a pCMV plasmid DNA) have been prepared by the present inventors. Perhaps even more unexpectedly, the increased adsorption levels of the polynucleotide-containing species were found to result in a corresponding increase in the immunogenicity that is observed upon injection into host animals (i.e., mice).

In many embodiments, the polynucleotide-containing species encodes a polypeptide-containing species such as a polypeptide-containing antigen. As a result, the microparticles of the present invention are particularly useful for immunization against intracellular viruses which normally elicit poor immune responses.

For example, the present invention will find use for stimulating an immune response against a wide variety of polypeptide-containing antigens from the herpesvirus family, including proteins derived from herpes simplex virus (HSV) types 1 and 2, such as HSV-1 and HSV-2 glycoproteins gB, gD and gH; antigens derived from varicella zoster virus (VZV), Epstein-Barr virus (EBV) and cytomegalovirus (CMV) including CMV gB and gH; and antigens derived from other human herpesviruses such as HHV6 and HHV7. (See, e.g. Chee et al., Cytomegaloviruses (J.K. McDougall, ed., Springer-Verlag 1990) pp. 125-169, for a review of the protein coding content of cytomegalovirus; McGeoch et al., J. Gen. Virol. (1988) 69:1531-1574, for a discussion of the various HSV-1 encoded proteins; U.S. Patent No. 5,171,568 for a discussion of HSV-1 and HSV-2 gB and gD proteins and the genes encoding therefor; Baer et al., Nature (1984) 310:207-211, for the identification of protein coding sequences in an EBV genome; and Davison and Scott, J. Gen. Virol. (1986) 67:1759-1816, for a review of VZV.)

Antigens from the hepatitis family of viruses, including hepatitis A virus (HAV), hepatitis B virus (HBV), hepatitis C virus (HCV), the delta hepatitis virus (HDV), hepatitis E virus (HEV) and hepatitis G virus (HGV), can also be conveniently used in the techniques described herein. By way of example, the viral genomic sequence of HCV is known, as are methods for obtaining the sequence. See, e.g., International Publication Nos. WO 89/04669; WO 90/11089; and WO 90/14436. The HCV genome encodes several viral proteins, including E1 (also known as E) and E2 (also known as E2/NSI) and an N-terminal nucleocapsid protein (termed "core") (see, Houghton et al., *Hepatology* (1991) 14:381-388, for a discussion of HCV proteins, including E1 and E2). Each of these proteins, as well as antigenic fragments thereof, will find use in the present composition and methods.

Similarly, the sequence for the δ-antigen from HDV is known (see, e.g., U.S. Patent No. 5,378,814) and this antigen can also be conveniently used in the present composition and methods. Additionally, antigens derived from HBV, such as the core antigen, the surface antigen, sAg, as well as the presurface sequences, pre-S1 and pre-S2 (formerly called pre-S), as well as combinations of the above, such as sAg/pre-S1, sAg/pre-S2, sAg/pre-S1/pre-S2, and pre-S1/pre-S2, will find use herein. See, e.g., "HBV Vaccines - from the laboratory to license: a case study" in Mackett, M. and Williamson, J.D., Human Vaccines and Vaccination, pp. 159-176, for a discussion of HBV structure; and U.S. Patent Nos. 4,722,840, 5,098,704, 5,324,513, incorporated herein by reference in their entireties; Beames et al., J. Virol. (1995) 69:6833-6838, Bimbaum et al., J. Virol. (1990) 64:3319-3330; and Zhou et al., J. Virol. (1991) 65:5457-5464.

Antigens derived from other viruses will also find use in the compositions and methods of the present invention, such as without limitation, proteins from members of the families Picornaviridae (e.g., polioviruses, etc.); Caliciviridae; Togaviridae (e.g., rubella virus, dengue virus, etc.); Flaviviridae; Coronaviridae; Reoviridae; Birnaviridae; Rhabodoviridae (e.g., rabies virus, etc.); Filoviridae; Paramyxoviridae (e.g., mumps virus, measles virus, respiratory syncytial virus, etc.); Orthomyxoviridae (e.g., influenza virus types A, B and C, etc.); Bunyaviridae; Arenaviridae; Retroviradae (e.g., HTLV-I; HTLV-II; HIV-1 (also known as HTLV-III, LAV, ARV, hTLR, etc.)), including but not limited to antigens from the isolates HIV_{IIIb}, HIV_{SF2}, HIV_{LAV}, HIV_{LAI}, HIV_{MN}); HIV-1_{CM235,} HIV-1_{US4}; HIV-2; simian immunodeficiency virus (SIV) among others. Additionally, antigens may also be derived from human papillomavirus (HPV) and the tick-borne encephalitis viruses. See, e.g. Virology, 3rd Edition (W.K. Joklik ed. 1988); Fundamental Virology, 2nd Edition (B.N. Fields and D.M. Knipe, eds. 1991), for a description of these and other viruses.

More particularly, the gp120 or gp140 envelope proteins from any of the above HIV isolates, including members of the various genetic subtypes of HIV, are known and reported (see, e.g., Myers et al., Los Alamos Database, Los Alamos National Laboratory, Los Alamos, New Mexico (1992); Myers et al., Human Retroviruses and Aids, 1990, Los Alamos, New Mexico: Los Alamos National Laboratory; and Modrow et al., J. Virol. (1987) 61:570-578, for a comparison of the envelope sequences of a variety of HIV isolates) and antigens derived from any of these isolates will find use in the present methods. Furthermore, the invention is equally applicable to other immunogenic proteins derived from any of the various HIV isolates, including any of the various envelope proteins such as gp160 and gp41, gag antigens such as p24gag and p55gag, as well as proteins derived from the pol and tat regions.

Influenza virus is another example of a virus for which the present invention will be particularly useful. Specifically, the envelope glycoproteins HA and NA of influenza A are of particular interest for generating an immune response. Numerous HA subtypes of influenza A have been identified (Kawaoka et al., Virology (1990) 179:759-767; Webster et al., "Antigenic variation among type A influenza viruses," p. 127-168. In: P. Palese and D.W. Kingsbury (ed.), Genetics of influenza viruses. Springer-Verlag, New York). Thus, proteins derived from any of these isolates can also be used in the compositions and methods described herein.

The compositions and methods described herein will also find use with numerous bacterial antigens, such as those derived from organisms that cause diphtheria, cholera, tuberculosis, anthrax, tetanus, pertussis, meningitis, and other pathogenic states, including, without limitation, *Bordetella pertussis*, *Neisseria meningitides* (for instance A, B, C, Y, W, e.g., W₁₃₅), *Neisseria gonorrhoeae*, *Helicobacter pylori*, and *Haemophilus influenza. Hemophilus influenza* type B (HIB), *Helicobacter pylori*, and combinations thereof. Examples of antigens from *Neisseria meningitides* B are disclosed in the following co-owned patent applications: PCT/US99/09346; PCT IB98/01665; and PCT IB99/00103. Examples of parasitic antigens include those derived from organisms causing malaria and Lyme disease.

Additional antigens for use with the invention, not necessarily exclusive of those listed elsewhere in this application, include the following: (a) a protein antigen from *N. meningitidis* serogroup B, such as those in Refs. 1 to 7 below; (b) an outer-membrane vesicle (OMV) preparation from *N. meningitidis* serogroup B, such as those disclosed in Refs. 8, 9, 10, 11, etc. below; (c) a saccharide antigen from *N. meningitidis* serogroup A, C, W135 and/or Y, such as the oligosaccharide disclosed in Ref. 12 below from serogroup C (see also Ref. 13); (d) a saccharide antigen from *Streptococcus pneumoniae* [e.g. Refs. 14, 15, 16]. (e) an antigen from *N. gonorrhoeae* [e.g., Refs. 1, 2, 3]; (e) an antigen from *Chlamydia pneumoniae* [e.g., Refs. 17, 18, 19, 20, 21, 22, 23]; (f) an antigen from *Chlamydia trachomatis* [e.g. Ref. 24]; (g) an antigen from hepatitis A virus, such as inactivated virus [e.g., Refs. 25, 26]; (h) an antigen from hepatitis B virus, such as the surface and/or core antigens [e.g., Refs. 26, 27]; (i) an antigen from hepatitis C virus [e.g. Ref. 28]; (j) an antigen from *Bordetella pertussis*, such as pertussis holotoxin (PT) and filamentous haemaglutinin (FHA) from *B. pertussis*, optionally also in combination with pertactin and/or agglutinogens 2 and 3 [e.g., Refs. 29 & 30]; (k) a diphtheria antigen, such as diphtheria toxoid [e.g., chapter 3 of Ref. 31] e.g. the CRM₁₉₇ mutant [e.g., Ref. 32]; (1) a tetanus antigen, such as a tetanus toxoid [e.g., chapter 4 of Ref. 31]; (m) a protein antigen from *Helicobacter pylori* such as CagA [e.g. Ref. 33], VacA [e.g. Ref. 33], NAP [e.g. Ref. 34], HopX [e.g. Ref. 35], HopY [e.g. Ref. 35] and/or urease; (n) a saccharide antigen from *Haemophilus influenzae* B [e.g. Ref. 13]; (o) an antigen from *Porphyramonas gingivalis* [e.g. Ref. 36]; (p) polio antigen(s) [e.g. Refs. 37, 38] such as IPV or OPV; (q) rabies antigen(s) [e.g. Ref. 39] such as lyophilized inactivated virus [e.g. Ref. 40, Rabavert™); (r) measles, mumps and/or rubella antigens [e.g., chapters 9, 10 and 11 of Ref. 31]; (s) influenza antigen(s) [e.g. chapter 19 of Ref. 31], such as the haemagglutinin and/or neuraminidase surface proteins; (t) an antigen from *Moraxella catarrhalis* [e.g., time 41]; (u) an antigen from *Streptococcus agalactiae* (Group B streptococcus) [e.g. Refs. 42, 43]; (v) an antigen from *Streptococcus pyogenes* (Group A streptococcus) [e.g. Refs. 43,44, 45]; (w) an antigen from *Staphylococcus aureus* [e.g. Ref. 46]; and (x) compositions comprising one or more of these antigens. Where a saccharide or carbohydrate antigen is used, it is preferably conjugated to a carrier protein in order to enhance immunogenicity [e.g. Refs. 47 to 56]. Preferred carrier proteins are bacterial toxins or toxoids, such as diphtheria or tetanus toxoids. The CRM₁₉₇ diphtheria toxoid is particularly preferred. Other suitable carrier proteins include *N. meningitidis* outer membrane protein [e.g. Ref. 57], synthetic peptides [e.g. Refs. 58, 59], heat shock proteins [e.g. Ref. 60], pertussis proteins [e.g. Refs. 61, 62], protein D from *H. Influenzae* [e.g. Ref. 63], toxin A or B from *C. difficile* [e.g. Ref. 64], etc. Where a mixture comprises capsular saccharides from both serogroups A and C, it is preferred that the ratio (w/w) of MenA saccharide:MenC saccharide is greater than 1 (e.g. 2:1, 3:1, 4:1, 5:1, 10:1 or higher). Saccharides from different serogroups of *N. meningitidis* may be conjugated to the same or different carrier proteins. Any suitable conjugation reaction can be used, with any suitable linker where necessary. Toxic protein antigens may be detoxified where necessary (e.g. detoxification of pertussis toxin by chemical and/or means [Ref. 30]. See: International patent application 99/24578 [Ref. 1]; International patent application WO99/36544 [Ref. 2]; International patent application WO99/57280 [Ref. 3]; International patent application WO00/22430 [Ref. 4]; Tettelin et al., (2000) Science 287:1809-1815 [Ref. 5]; International patent application WO96/29412 [Ref. 6]; Pizza el al. (2000) Science 287:1816-1820 [Ref. 7]; International patent application PCT/IB01/00166 [Ref. 8]; Bjune et al. (1991) Lancet 338(8775):1093-1096 [Ref. 9]; Fukasawa et al. (1990) Vaccine 17:2951-2958 [Ref. 10]; Rosenqvist et al. (1998) Dev. Biol. Stand. 92:323-333 [Ref. 11]; Costantino et al. (1992) Vaccine 10:691-698 [Ref. 12]; Costantino et al. (1999) Vaccine 17:1251-1263 [Ref. 13]; Watson (2000) Padiatr Infect Dis J 19:331-332 [Ref. 14]; Rubin (2000) Pediatr Clin North Am 47:269-285, v [Ref. 15]; Jedrzejas (2001) Microbiol Mol Biol Rev 65:187-207 [Ref. 16]; International patent application filed on 3rd July 2001 claiming priority from GB-0016363.4 [Ref. 17]; Kalman et al. (1999) Nature Genetics 21 :385-389 [Ref. 18]; Read et al. (2000) Nucleic Acids Res 28:1397-406 [Ref. 19]; Shirai et al. (2000) J. Infect. Dis. 181(Suppl 3):S524-S527 [Ref. 20]; International patent application WO99/27105 [Ref. 21]; International patent application WO00/27994 [Ref. 22]; International patent application WO00/37494 [Ref. 23]; International patent application WO99/28475 [Ref. 24]; Bell (2000) Pediatr Infect Dis J 19:1187-1188 [Ref. 25]; Iwarson (1995) APMIS 103:321-326 [Ref. 26]; Gerlich et al. (1990) Vaccine 8 Suppl:S63-68 & 79-80 [Ref. 27]; Hsu et al. (1999) Clin Liver Dis 3:901-915 [Ref. 28]; Gustafsson et al. (1996) N. Engl. J. Med. 334:349-355 [Ref. 29]; Rappuoli et al. (1991) TIBTECH 9:232-238 [Ref. 30]; Vaccines (1988) eds. Plotkin & Mortimer. ISBN 0-7216-1946-0 [Ref. 31]; Del Guidice et al. (1998) Molecular Aspects of Medicine 19:1-70 [Ref. 32]; International patent application WO93/18150 [Ref. 33]; International patent application WO99/53310 [Ref. 34]; International patent application WO98/04702 [Ref. 35]; Ross et al. (2001) Vaccine 19:4135-4142 [Ref. 36]; Sutter et al. (2000) Pediatr Clin North Am 47:287-308 [Ref. 37]; Zimmerman & Spann (1999) Am Fam Physician 59:113-118, 125-126 [Ref. 38]; Dreesen (1997) Vaccine 15 Suppl:S2-6 [Ref. 39]; MMWR Morb Mortal Wkly Rep 1998 Jan 16;47(1):12, 19 [Ref. 40]; McMichael (2000) Vaccine 19 Suppl 1:S101-107 [Ref. 41]; Schuchat (1999) Lancet 353(9146):51-6 [Ref. 42]; GB patent applications 0026333.5, 0028727.6 & 0105640.7 [Ref. 43]; Dale (1999) Infect Dis Clin North Am 13:227-43, viii [Ref. 44]; Ferretti et al. (2001) PNAS USA 98:4658-4663 [Ref. 45]; Kuroda et al. (2001) Lancet 357(9264):1225-1240; see also pages 1218-1219 [Ref. 46]; Ramsay et al. (2001) Lancet 357(9251):195-196 [Ref. 47]; Lindberg (1999) Vaccine 17 Suppl 2:S28-36 [Ref. 48]; Buttery & Moxon (2000) JR Coll Physicians London 34:163-168 [Ref. 49]; Ahmad & Chapnick (1999) Infect Dis Clin North Am 13:113-133, vii [Ref. 50]; Goldblatt (1998) J. Med. Microbiol. 47:563-567 [Ref. 51]; European patent 0 477 508 [Ref. 52]; US Patent No. 5,306,492 [Ref. 53]; International patent application WO98/42721 [Ref. 54]; Conjugate Vaccines (eds. Cruse et al.) ISBN 3805549326, particularly vol. 10:48-114 [Ref. 55]; Hermanson (1996) Bioconjugate Techniques ISBN: 0123423368 & 012342335X [Ref. 56]; European patent application 0372501 [Ref. 57]; European patent application 0378881 [Ref. 58]; European patent application 0427347 [Ref. 59]; International patent application WO93/17712 [Ref. 60]; International patent application WO98/58668 [Ref. 61]; European patent application 0471177 [Ref. 62]; International patent application WO00/56360 [Ref. 63]; international patent application WO00/61761 [Ref. 64].

Where diphtheria antigen is included in the composition it is preferred also to include tetanus antigen and pertussis antigens. Similarly, where a tetanus antigen is included it is preferred also to include diphtheria and pertussis antigens. Similarly, where a pertussis antigen is included it is preferred also to include diphtheria and tetanus antigens.

Additional antigens include antigens directed to plague, Rocky Mountain spotted fever, smallpox, typhoid, typhus, feline leukemia virus, and yellow fever.

The microparticles of the present invention can be used to deliver a wide variety of species in addition to surface-adsorbed polynucleotide-containing species. Such additional species include: (a) antigens, such as the above polypeptide-containing antigens, (b) pharmaceuticals such as antibiotics and antiviral agents, nonsteroidal antiinflammatory drugs, analgesics, vasodilators, cardiovascular drugs, psychotropics, neuroleptics, antidepressants, antiparkinson drugs, beta blockers, calcium channel blockers, bradykinin inhibitors, ACE-inhibitors, vasodilators, prolactin inhibitors, steroids, hormone antagonists, antihistamines, serotonin antagonists, heparin, chemotherapeutic agents, antineoplastics and growth factors, including but not limited to PDGF, EGF, KGF, IGF-1 and IGF-2, FGF, (c) hormones including peptide hormones such as insulin, proinsulin, growth hormone, GHRH, LHRH, EGF, somatostatin, SNX-111, BNP, insulinotropin, ANP, FSH, LH, PSH and hCG, gonadal steroid hormones (androgens, estrogens and progesterone), thyroid-stimulating hormone, inhibin, cholecystokinin, ACTH, CRF, dynorphins, endorphins, endothelin, fibronectin fragments, galanin, gastrin, insulinotropin, glucagon, GTP-binding protein fragments, guanylin, the leukokinins, magainin, mastoparans, dermaseptin, systemin, neuromedins, neurotensin, pancreastatin, pancreatic polypeptide, substance P, secretin, thymosin, and the like, (d) enzymes, (e) transcription or translation mediators, (f) intermediates in metabolic pathways, (g) immunomodulators, such as any of the various cytokines including interleukin-1, interleukin-2, interleukin-3, interleukin-4, and gamma-interferon, and (h) adjuvants (see below).

Such additional species can be, for example, adsorbed on the surfaces of the microparticles along with the polynucleotide-containing species, entrapped within the microparticles, dissolved or dispersed in solution but unbound to the microparticles, and/or adsorbed to or entrapped within another group of microparticles.

In some embodiments, the microparticle compositions of the present invention can be used for site-specific targeted delivery. For example, intravenous administration of the microparticle compositions can be used for targeting the lung, liver, spleen, blood circulation, or bone marrow.

Biodegradable polymers for manufacturing microparticles for use with the present invention are readily commercially available from, e.g., Boehringer Ingelheim, Germany and Birmingham Polymers, Inc., Birmingham, AL. For example, useful polymers for forming the microparticles herein include homopolymers, copolymers and polymer blends derived from the following: polyhydroxybutyric acid (also known as polyhydroxybutyrate); polyhydroxy valeric acid (also known as polyhydroxyvalerate); polyglycolic acid (PGA) (also known as polyglycolide): polylactic acid (PLA) (also known as polylactide); polydioxanone; polycaprolactone; polyorthoester; and polyanhydride. More preferred are poly(α-hydroxy acids), such as poly(L-lactide), poly(D,L-lactide) (both known as "PLA" herein), poly(hydoxybutyrates), copolymers of lactide and glycolide, such as poly(D,L-lactide-co-glycolide) (designated as "PLG" herein) or copolymers of D,L-lactide and caprolactone. Particularly preferred polymers for use herein are PLA and PLG polymers.

These polymers are available in a variety of molecular weights, and the appropriate molecular weight for a given use is readily determined by one of skill in the art. Thus, e.g., for PLA, a suitable molecular weight will be on the order of about 2000 to 5000. For PLG, suitable molecular weights will generally range from about 10,000 to about 200,000, typically about 15,000 to about 150,000.

If a copolymer is used, polymers with a variety of monomer ratios may be available. For example, where PLG is used to form the microparticles, a variety of lactide:glycolide molar ratios will find use herein and the ratio is largely a matter of choice, depending in part on the coadministered adsorbed/entrapped species and the rate of degradation desired. For example, a 50:50 PLG polymer, containing 50% D,L-lactide and 50% glycolide, will provide a fast resorbing copolymer while 75:25 PLG degrades more slowly, and 85:15 and 90:10, even more slowly, due to the increased lactide component. It is readily apparent that a suitable ratio of lactide:glycolide is easily determined by one of skill in the art based, for example, on the nature of the antigen and disorder in question. Moreover, mixtures of microparticles with varying lactide:glycolide ratios may find use herein in order to achieve the desired release kinetics. Degradation rate of the microparticles of the present invention can also be controlled by such factors as polymer molecular weight and polymer crystallinity. PLG copolymers with varying lactide:glycolide ratios and molecular weights are readily available commercially from a number of sources including from Boehringer Ingelheim, Germany and Birmingham Polymers, Inc., Birmingham, AL. Some exemplary PLG copolymers include: (a) RG 502, a PLG having a 50:50 lactide/glycolide molar ratio and a molecular weight of 12,000 Da; (b) RG 503, a PLG having a 50:50 lactide/glycolide molar ratio and a molecular weight of 34,000 Da; (c) RG 504, a PLG having a 50:50 lactide/glycolide molar ratio and a molecular weight of 48,000 Da, (d) RG 752, a PLG having a 75:25 lactide/glycolide molar ratio and a molecular weight of 22,000 Da; and (e) RG 755, a PLG having a 75:25 lactide/glycolide molar ratio and a molecular weight of 68,000 Da. PLG polymers can also be synthesized by simple polycondensation of the lactic acid component using techniques well known in the art, such as described in Tabata et al., J. Biomed. Mater. Res. (1988) 22:837-858.

Where used, poly(D,L-lactide-co-glycolide) polymers are typically those having a molar lactide/glycolide molar ratio ranging from 20:80 to 80:20, typically 40:60 to 60:40, and having a molecular weight ranging from 10,000 to 100,000 Daltons, typically from 20,000 Daltons to 70,000 Daltons.

The microparticles are prepared using any of several methods well known in the art. For example, in some embodiments, double emulsion/solvent evaporation techniques, such as those described in U.S. Patent No. 3,523,907 and Ogawa et al., Chem. Pharm. Bull. (1988) 36:1095-1103, can be used herein to make the microparticles. These techniques involve the formation of a primary emulsion consisting of droplets of polymer solution, which is subsequently mixed with a continuous aqueous phase containing a particle stabilizer/ surfactant.

In other embodiments, microparticles can also be formed using spray-drying and coacervation as described in, e.g., Thomasin et al., J. Controlled Release (1996) 41:131; U.S. Patent No. 2,800,457; Masters, K. (1976) Spray Drying 2nd Ed. Wiley, New York; air-suspension coating techniques, such as pan coating and Wurster coating, as described by Hall et al., (1980) The "Wurster Process" in Controlled Release Technologies: Methods, Theory, and Applications (A.F. Kydonieus, ed.), Vol. 2, pp. 133-154 CRC Press, Boca Raton, Florida and Deasy, P.B., Crit. Rev. Ther. Drug Carrier Syst. (1988) S(2):99-139; and ionic gelation as described by, e.g., Lim et al., Science (1980) 210:908-910.

In preferred embodiments, a water-in-oil-in-water (w/o/w) solvent evaporation system can be used to form the microparticles, along the lines described by O'Hagan et al., Vaccine (1993) 11:965-969, PCT/US99/17308 (WO 00/06123) to O'Hagan et al. and Jeffery et al., Pharm. Res. (1993) 10:362.

In general, a polymer of interest such as PLG is dissolved in an organic solvent, such as ethyl acetate, dimethylchloride (also called methylene chloride and dichloromethane), acetonitrile, acetone, chloroform, and the like. The polymer will be provided in about a 1-30%, preferably about a 2-15%, more preferably about a 3-10% and most preferably, about a 4-8% solution, in organic solvent. The polymer solution is then combined with a first volume of aqueous solution and emulsified to form an o/w emulsion. The aqueous solution can be, for example, deionized water, normal saline, or a buffered solution such as phosphate-buffered saline (PBS) or a sodium citrate/ethylenediaminetetraacetic acid (sodium citrate/ETDA) buffer solution. The latter solutions can (a) provide a tonicity, i.e., osmolality, that is essentially the same as normal physiological fluids and (b) maintain a pH compatible with normal physiological conditions. Alternatively, the tonicity and/or pH characteristics of the compositions of the present invention can be adjusted after microparticle formation and prior to administration. Where one or more species are to be entrapped within the microparticles, the species can be added to either the polymer solution or the aqueous solution. Preferably, the volume ratio of polymer solution to aqueous solution ranges from about 5:1 to about 20:1, more preferably about 10:1. Emulsification is conducted using any equipment appropriate for this task, and is typically a high-shear device such as, e.g., a homogenizer.

In some embodiments, one or more components are entrapped within the microparticles. For example, the component(s) can be introduced by adding the same: (a) to the polymer solution, if in oil-soluble or oil-dispersible form, or (b) to the aqueous solution, if in water-soluble or water-dispersible form.

A volume of the o/w emulsion is then preferably combined with a larger second volume of an aqueous solution, which typically contains a surfactant. The volume ratio of aqueous solution to o/w emulsion typically ranges from about 2:1 to 10:1, more typically about 4:1. Examples of surfactants appropriate for the practice of the invention are listed above. Those of ordinary skill in the art may readily select surfactants appropriate for the type of species to be adsorbed. For example, microparticles manufactured in the presence of charged surfactants, such as anionic or cationic surfactants, may yield microparticles with a surface having a net negative or a net positive charge, which can adsorb a wide variety of molecules. For example, microparticles manufactured with anionic surfactants, such as sodium dodecyl sulfate (SDS), e.g., SDS-PLG microparticles, adsorb positively charged species, for example, polypeptide-containing species such as proteins. Similarly, microparticles manufactured with cationic surfactants, such as CTAB, e.g., PLG/CTAB microparticles, adsorb negatively charged species, for example, polynucleotide-containing species such as DNA. Where the species to be adsorbed have regions of positive and negative charge, either cationic or anionic or nonionic surfactants may be appropriate. In the present invention, cationic surfactants are preferred. Certain species may adsorb more readily to microparticles having a combination of surfactants. Moreover, in some instances, it may be desirable to add surfactant to the above organic solution.

Where a cationic surfactant such as CTAB is used, it is typically provided in about a 0.00025-1 % solution, more typically about a 0.0025-0.1 % solution. Generally, a weight-to-weight surfactant-to-polymer ratio in the range of from about 0.0001:1 to about 0.5:1, more typically from about 0.001:1 to about 0.1:1, and even more typically from about 0.0025:1 to about 0.05:1 is used.

The mixture is then homogenized to produce a stable w/o/w double emulsion. Each of the above homogenization steps is typically conducted at a room temperature (i.e., 25°C) or less, more typically less, for example, while cooling within an ice bath.

Organic solvents are then evaporated.

The formulation parameters can be manipulated to allow the preparation of small microparticles on the order of 0.05 µm (50 nm) to larger microparticles 50 µm or even larger. See, e.g., Jeffery et al., Pharm. Res. (1993) 10:362-368; McGee et al., J. Microencap. (1996). For example, reduced agitation results in larger microparticles, as does an increase in internal phase volume and an increase in polymer concentration. Small particles are produced by increased agitation as well as low aqueous phase volumes, high concentrations of emulsion stabilizers and a decrease in polymer concentration.

Particle size can be determined by, e.g., laser light scattering, using for example, a spectrometer incorporating a helium-neon laser. Generally, particle size is determined at room temperature and involves multiple analyses of the sample in question (e.g., 5-10 times) to yield an average value for the particle diameter. Particle size is also readily determined using scanning electron microscopy (SEM).

Following preparation, microparticles can be stored as is or lyophilized for future use. In order to adsorb the desired species to the microparticles, the microparticle preparation can be simply mixed with the species of interest and the resulting formulation can be lyophilized prior to use if desired. The content of the adsorbed species can be determined using standard techniques.

For example, polynucleotide-containing species can be added to the microparticles to yield microparticles with adsorbed polynucleotide-containing species having a weight-to-weight ratio of typically 0.1:1 to 0.25:1.

The polymer microparticles of the present invention may have a variety of species entrapped or encapsulated within them, as well as having a variety of species adsorbed thereon. Thus, for example, one of skill in the art may prepare in accordance with the invention microparticles having adsorbed adjuvants and/or adsorbed polypeptide antigens, in addition to adsorbed polynucleotide-containing species. One of skill in the art may also prepare in accordance with the invention microparticles having, for example, encapsulated adjuvants, encapsulated polypeptide antigens and/or encapsulated polynucleotide-containing species.

Once the microparticles with adsorbed species are produced, they are formulated into pharmaceutical compositions, including vaccines, to treat and/or diagnose a wide variety of disorders. The compositions will generally include one or more pharmaceutically acceptable excipients. For example, vehicles such as water, saline, glycerol, polyethylene glycol, hyaluronic acid, ethanol, etc. may be used. Other excipients, such as wetting or emulsifying agents, biological buffering substances, and the like, may be present in such vehicles. A biological buffer can be virtually any solution which is pharmacologically acceptable and which provides the formulation with the desired pH, i.e., a pH in the physiological range. Examples of solutions include saline, phosphate buffered saline, Tris buffered saline, Hank's buffered saline, and the like. Other excipients known in the art can also be introduced into the final dosage form, including binders, disintegrants, fillers (diluents), lubricants, glidants (flow enhancers), compression aids, colors, sweeteners, preservatives, suspensing/dispersing agents, film formers/coatings, flavors and printing inks.

Adjuvants maybe used to enhance the effectiveness of the microparticle compositions. The adjuvants may be administered concurrently with the microparticles of the present invention, e.g., in the same composition or in separate compositions. Alternatively, an adjuvant may be administered prior or subsequent to the microparticle compositions of the present invention. In some embodiments, the adjuvant, such as an immunological adjuvant, is encapsulated in the microparticle. Alternatively, the adjuvant may be adsorbed on the microparticle.

Immunological adjuvants include, but are not limited to: (1) aluminum salts (alum), such as aluminum hydroxide, aluminum phosphate, aluminum sulfate, etc.; (2) oil-in water emulsion formulations (with or without other specific immunostimulating agents such as muramyl peptides (see below) or bacterial cell wall components), such as for example (a) MF59 (International Publication No. WO90/14837; Chapter 10 in Vaccine design: the subunit and adjuvant approach, eds. Powell & Newman, Plenum Press 1995), containing 5% Squalene, 0.5% Tween 80, and 0.5% Span 85 (optionally containing various amounts of MTP-PE (see below), although not required) formulated into submicron particles using a microfluidizer such as Model 110Y microfluidizer (Microfluidics, Newton, MA), (b) SAF, containing 10% Squalane, 0.4% Tween 80, 5% pluronic-blocked polymer L121, and thr-MDP (see below) either microfluidized into a submicron emulsion or vortexed to generate a larger particle size emulsion, and (c) Ribi™ adjuvant system (RAS), (Ribi Immunochem, Hamilton, MT) containing 2% Squalene, 0.2% Tween 80, and one or more bacterial cell wall components from the group consisting of monophosphorylipid A (MPL), trehalose dimycolate (TDM), and cell wall skeleton (CWS), preferably MPL + CWS (Detox™) (for a further discussion of suitable submicron oil-in-water emulsions for use herein, see commonly owned, patent application no. 09/015,736, filed on January 29, 1998); (3) saponin adjuvants, such as Quil A, or QS21 (e.g., Stimulon™ (Cambridge Bioscience, Worcester, MA)) maybe used or particles generated therefrom such as ISCOMs (immunostimulating complexes), which ICOMS maybe devoid of additional detergent e.g., WO00/07621; (4) Complete Freunds Adjuvant (CFA) and Incomplete Freunds Adjuvant (IFA); (5) cytokines, such as interleukins (e.g. IL-1, IL-2, IL-4, IL-5, IL-6, IL-7, IL-12 (WO99/44636), etc.), interferons (e.g. gamma interferon), macrophage colony stimulating factor (M-CSF), tumor necrosis factor (TNF), etc.; (6) phospholipids, e.g., monophosphoryl lipid A compounds, including monophosphoryl lipid A (MPL) and its derivatives, such as 3-O-deacylated MPL (3dMPL), e.g., GB-2220221, EP-A-0689454, optionally in the substantial absence of alum when used with pneumococcal saccharides, e.g., WO00/56358; (7) combinations of 3dMPL with, for example, QS21 and/or oil-in-water emulsions, e.g., EP-A-0835318, EP-A-0735898, EP-A-0761231; (8) oligonucleotides comprising CpG motifs, described above; (9) a polyoxyethylene ether or a polyoxyethylene ester e.g. WO99/52549; (10) a polyoxyethylene sorbitan ester surfactant in combination with an octoxynol (WO01/21207) or a polyoxyethylene alkyl ether or ester surfactant in combination with at least one additional non-ionic surfactant such as an octoxynol (WO01/21152); (11) a saponin and an immunostimulatory oligonucleotide (e.g., a CpG oligonucleotide) (WO00/62800); (12) an immunostimulant and a particle of metal salt e.g. WO00/23105; (13) a saponin and an oil-in-water emulsion, e.g., WO99/11241; (14) a saponin (e.g. QS21) + 3dMPL + IL-12 (optionally + a sterol), e.g., WO98/57659; (15) detoxified mutants of a bacterial ADP-ribosylating toxin such as a cholera toxin (CT), a pertussis toxin (PT), or an *E. coli* heat-labile toxin (LT), particularly LT-K63 (where lysine is substituted for the wild-type amino acid at position 63), LT-R72 (where arginine is substituted for the wild-type amino acid at position 72), CT-S109 (where serine is substituted for the wild-type amino acid at position 109), and PT-K9/G129 (where lysine is substituted for the wild-type amino acid at position 9 and glycine substituted at position 129) (see, e.g., International Publication Nos. WO93/13202 and WO92/19265); (16) adjuvants comprising dsRNA, described above; and (17) other substances that act as immunostimulating agents to enhance the effectiveness of the composition.

Muramyl peptides include, but are not limited to, N-acetyl-muramyl-L-threonyl-D-isoglutamine (thr-MDP), N-acteyl-normuramyl-L-alanyl-D-isogluatme (nor-MDP), N-acetylmuramyl-L-alanyl-D-isogluatminyl-L-alanine-2-(1'-2'-dipalmitoyl-*sn-*glycero-3-huydroxyphosphoryloxy)-ethylamine (MTP-PE), etc.

For additional examples of adjuvants, see Vaccine Design, The Subunit and the Adjuvant Approach, Powell, M.F. and Newman, M.J, eds., Plenum Press, 1995).

Once formulated, the compositions of the invention can be administered parenterally, e.g., by injection (which may be needleless). The compositions can be injected subcutaneously, intraperitoneally, intravenously, intraarterially, intradermally, or intramuscularly. Other modes of administration include nasal, mucosal, intraoccular, rectal, vaginal, oral and pulmonary administration, suppositories, and transdermal or transcutaneous applications.

Dosage treatment may be a single dose schedule or a multiple dose schedule. A multiple dose schedule is one in which a primary course of administration may be given, for example, with 1-10 separate doses, followed by other doses given at subsequent time intervals, chosen to maintain and/or reinforce the therapeutic response, for example at 1-4 months for a second dose, and if needed, a subsequent dose(s) after several months. The dosage regimen will also be, at least in part, determined by the need of the subject and be dependent on the judgment of the practitioner.

Furthermore, if prevention of disease is desired, the microparticles are generally administered prior to primary infection with the pathogen of interest. If treatment is desired, e.g., the reduction of symptoms or recurrences, the microparticles are generally administered subsequent to primary infection.

### C. Experimental

Below are examples of specific embodiments for carrying out the present invention. The examples are offered for illustrative purposes only, and are not intended to limit the scope of the present invention in any way.

Efforts have been made to ensure accuracy with respect to numbers used (e.g., amounts, temperatures, etc.), but some experimental error and deviation should, of course, be allowed for.

### Example 1

### Preparation of Microparticles

16.6 ml of 6 w/v% RG504 (a PLG Polymer having a 50:50 lactide/glycolide molar ratio and a molecular weight of 42-45 kDaltons, available from Boehringer Ingelheim) in dimethyl chloride is emulsified, in an ice bath, with 1.5 ml TE buffer using the 10mm probe of an Omni benchtop homogenizer for 3 minutes at 10,000 rpm. To this primary o/w emulsion is added 70 ml of a distilled water solution containing 10 mg CTAB (Sigma Chemical Co., St. Louis, MO), followed by homogenization for 15 minutes at 10,000 rpm, also in an ice bath. This results in the formation of a w/o/w emulsion, which is subsequently stirred with a magnetic stirrer overnight, allowing the methylene chloride to evaporate. After overnight stirring, 72 ml of a suspension remains, which contains 1 g of PLG (14 mg PLG/ml) and 10 mg CTAB (.14 mg CTAB/ml), or 1% CTAB relative to PLG (referred to herein as a "1% CTAB suspension").

This procedure is repeated, except that 40 mg CTAB are provided in the distilled water solution. This produces a suspension that contains 1 g of PLG (14 mg PLG/ml) and 40 mg CTAB (0.56 mg PLG/ml), or 4% CTAB vis-à-vis the PLG (referred to herein as a "4% CTAB suspension").

### Example 2

### Absorption of DNA on the Surface of Microparticles

2 mg/ml and 4 mg/ml solutions of DNA in 1x TE buffer are prepared. In this example the DNA is a pCMVgag plasmid encoding HIV p55 gag protein under the control of the cytomegalovirus early promoter. A first mixture (referred to as "1% CTAB, 4% DNA") is prepared by combining 7.15 ml of the 1% CTAB suspension (which contains 100 mg PLG) with 2 ml of the 2 mg/ml DNA solution (which contains 4 mg DNA). Adsorption is allowed to proceed by gently stirring with a magnetic stirrer for 6 hours at 4°C, followed by lyophilization.

A second mixture (referred to as "1% CTAB, 8% DNA") is prepared by combining 7.15 ml of the 1% CTAB suspension (which contains 100 mg PLG) with 2 ml of the 4 mg/ml DNA solution (which contains 8 mg DNA), followed by stirring and lyophilization.

Additional mixtures are created as indicated in the Table 1 below.

**Table 1.**

| | **1% CTAB Suspension** | **4% CTAB Suspension** | **2 mg/ml DNA Solution** | **4 mg/ml DNA Solution** |
|---|---|---|---|---|
| 1% CTAB, 4% DNA | 7.15 ml | | 2 ml | |
| 1% CTAB, 8% DNA | 7.15 ml | | | 2 ml |
| 1% CTAB, 12% DNA | 7.15 ml | | | 3 ml |
| 1% CTAB, 16% DNA | 7.15 ml | | | 4 ml |
| 1% CTAB, 20% DNA | 7.15 ml | | | 5 ml |
| 4% CTAB, 4% DNA | | 7.15 ml | 2 ml | |
| 4% CTAB, 8% DNA | | 7.15 ml | | 2 ml |
| 4% CTAB, 12% DNA | | 7.15 ml | | 3 ml |
| 4% CTAB, 16% DNA | | 7.15 ml | | 4 ml |
| 4% CTAB, 20% DNA | | 7.15 ml | | 5 ml |

The microparticles are sized in a Malvern Master sizer after lyophilization. Sizes were measured to be 15 microns or less.

### Example 3

### Quantification of DNA on the Surface of Microparticles

The total DNA content was analyzed by resuspending 10 mg of the lyophilized microparticles 0.2N NaOH and reading the clear solution after hydrolysis at 260 nm. Results are given in Table 2 below. In vitro DNA release was analyzed by resuspending 10 mg of the lyophilized microparticles in 1 ml of PBS and rocking at 37°C. The supernatant was assayed after 24 hours for DNA content by reading the absorbance at 260 nm.

The amount of DNA adsorbed (loaded) on the microparticles after 24 hours is calculated by subtracting the amount of DNA in the supernatant from the total DNA content. The loading efficiency was calculated based on the amount of DNA adsorbed relative to the DNA that is added to the CTAB suspension (referred to as the "target DNA"). Results are given in Table 2 below.

**Table 2.**

| | **Target DNA Load (wt%)** | **Loading Efficiency (%)** |
|---|---|---|
| 1% CTAB, 4% DNA | 4 | 76 |
| 1% CTAB, 8% DNA | 8 | 72 |
| 1% CTAB, 12% DNA | 12 | 68 |
| 1% CTAB, 16% DNA | 16 | 54 |
| 1% CTAB, 20% DNA | 20 | 54 |
| 4% CTAB, 4% DNA | 4 | 88 |
| 4% CTAB, 8% DNA | 8 | 78 |
| 4% CTAB, 12% DNA | 12 | 76 |
| 4% CTAB, 16% DNA | 16 | 64 |
| 4% CTAB, 20% DNA | 20 | 62 |

### Example 4

### Immunization Protocol

Injectable DNA formulations are prepared by suspending lyophilized microparticles from each of the above groups (with the specific amount from each group being the amount that is required to provide 10 µg DNA) in 0.1 ml of water for injection. The injectable DNA formulation is then injected intramuscularly in Balb-C mice. 10 µg of DNA alone is also injected into the mice as a control. Each formulation is injected into 10 mice. The mice were boosted after four weeks.

### Example 5

### Immunoassay

Two weeks after the second immunization (six weeks total), heparinized blood is collected and plasma was recovered by centrifugation. Anti-HIV antibodies were measured by enzyme-linked immunosorbent assay (ELISA) as follows. Wells of microtiter plates were coated with recombinant HIV-1.SF2 p55gag protein at 5 microgram/ml in PBS, 50 microliters per well, and incubated at 4°C overnight. The plates were washed six times with wash buffer (PBS, 0.3% Tween 20) and blocked at 37°C for 1 h with 200 microliters per well of blocking buffer (PBS, 0.3% Tween 20, 5% goat serum). Test samples were diluted 1:25 and then serially diluted threefold in blocking buffer. The block solution was aspirated, and then the plates were incubated at room temperature for 1 h with 70 microliters per well of each plasma dilution. After being washed six times, the plates were incubated for 1 h at 37°C with horseradish peroxidase-conjugated anti-IgG (1:8,000 dilution). Following six washes, the plates were developed with TMB substrate for 15 minutes. The reaction was stopped with 2N HCl and the optical densities (OD) measured at a wavelength of 450 nm. The titer was calculated to be the reciprocal of the dilution at which an OD₄₅₀ₙₘ of 0.5 was achieved.

The results are summarized in Table 3 to follow:

**Table 3.**

| **Formulation** | **GMT** | **Upper** | **Lower** |
|---|---|---|---|
| PLG/1% CTAB, 4% p55 DNA, 10 µg | 2011 | 1,582 | 2,556 |
| PLG/1% CTAB, 8% p55 DNA, 10 µg | 1100 | 522 | 2,318 |
| PLG/1% CTAB, 12% p55 DNA, 10 µg | 1328 | 963 | 1,831 |
| PLG/1% CTAB, 16% p55 DNA, 10 µg | 2517 | 1,908 | 3,321 |
| PLG/1% CTAB, 20% p55 DNA, 10 µg | 2341 | 1,822 | 3,007 |
| PLG/4% CTAB, 4% p55 DNA, 10 µg | 143 | 67 | 308 |
| PLG/4% CTAB, 8% p55 DNA, 10 µg | 765 | 536 | 1,094 |
| PLG/4% CTAB, 12% p55 DNA, 10 µg | 292 | 217 | 391 |
| PLG/4% CTAB, 16% p55 DNA, 10 µg | 443 | 345 | 568 |
| PLG/4% CTAB, 20% p55 DNA, 10 µg | 525 | 276 | 1,001 |
| HIV p55 DNA, 10 µg | 343 | 191 | 617 |

As can be seen from the above table, the PLG-CTAB microparticles with the adsorbed DNA induced significantly enhanced antibody titers in mice over naked DNA. Moreover, for each DNA target load (4%, 8%, 12%, 16% and 20%), 1% CTAB PLG microparticles displayed significantly enhanced antibody titers in mice relative to 4% CTAB PLG microparticles.

### Example 6

### CTL Assay

Spleens from immunized mice were also harvested at 6 weeks and a standard enzyme-linked immunospot (ELISPOT) assay was carried out. Briefly, single-cell suspensions from spleen are prepared and the concentration is adjusted to 3 X 10⁷ cells/ml. 100 µl of cell suspension are added to the first row of 96-well PVDF (polyvinylidene difluoride) plates, which have previously been coated overnight with rat anti-mouse IFN-γ (Pharmingen). After incubation overnight at 37°C, the plates are washed, and biotinylated anti-IFN-γ (Pharmingen) is added. After the plates are incubated at room temperature for 2 h and washed, avidin-peroxidase (Pharmingen) is added, and the plates are incubated for 30 min at 37°C and washed. The plates are developed with aminoethyl carbazole solution (Sigma) for 30 min. Color development is stopped by washing in tap water. Spots are counted in a Zeiss ELISpot reader.

Data are presented in the following Table 4.

**Table 4.**

| | **GMT** | **SE** |
|---|---|---|
| 1% CTAB, 4% DNA, 10 µg | 564 | 110 |
| 1% CTAB, 8% DNA, 10 µg | 633 | 103 |
| 1% CTAB, 12% DNA, 10 µg | 1077 | 278 |
| 1% CTAB, 16% DNA, 10 µg | 1576 | 152 |
| 1% CTAB, 20% DNA, 10 µg | 782 | 352 |
| 4% CTAB, 4% DNA, 10 µg | -- | -- |
| 4% CTAB, 8% DNA, 10 µg | 264 | 169 |
| 4% CTAB, 12% DNA, 10 µg | 731 | 190 |
| 4% CTAB, 16% DNA, 10 µg | 517 | 99 |
| 4% CTAB, 20% DNA, 10 µg | 507 | 160 |
| HIV p55 DNA, 10 µg | 498 | 230 |

As can be seen from the above table, the PLG-CTAB microparticles with the adsorbed DNA resulted in enhanced CTL induction in mice relative to naked DNA. Moreover, for all DNA target loads (4%, 8%, 12%, 16% and 20%), 1% CTAB PLG microparticles displayed significantly enhanced CTL induction, relative to 4% CTAB PLG microparticles.

## Claims

1. Microparticles comprising: (a) a biodegradable polymer selected from a poly(alpha-hydroxy acid), a polyhydroxy butyric acid, a polycaprolactone, a polyorthoester, a polyanhydride and a polycyanoacrylate; (b) a cationic surfactant; and (c) a first polynucleotide-containing molecule adsorbed on the surface of the microparticles, wherein the adsorbed first polynucleotide-containing molecule constitutes between 10 and 30 percent of the total weight of the microparticles.

2. The microparticles of claim 1, wherein the cationic surfactant comprises cetyltrimethylammonium bromide.

3. The microparticles of claim 1 or claim 2, wherein the microparticles have a diameter between 200 nanometers and 20 microns.

4. The microparticles of any one of claims 1-3, wherein the polymer comprises a polyhydroxy butyric acid, a polycaprolactone, a polyorthoester, a polyanhydride, or a polycyanoacrylate.

5. The microparticles of any one of claims 1-3, wherein the polymer comprises a poly(α-hydroxy acid).

6. The microparticles of claim 5, wherein the polymer comprises a poly(α-hydroxy acid) selected from poly(L-lactide), poly(D,L-lactide) and poly(lactide-co-glycolide).

7. The microparticles of any one of claims 1-6, wherein the first polynucleotide-containing molecule is an immunological adjuvant, comprises a CpG oligonucleotide or dsRNA, encodes a polypeptide-containing antigen, or is a vector construct that encodes a polypeptide-containing antigen.

8. The microparticles of claim 7, wherein the vector construct is an RNA vector construct.

9. The microparticles of claim 7, wherein the vector construct is a DNA vector construct.

10. The microparticles of claim 9, wherein the DNA vector construct is a plasmid.

11. The microparticles of claim 10, wherein the plasmid is pCMV.

12. The microparticles of claim 9, wherein the DNA vector construct is a RNA-virus-based plasmid.

13. The microparticles of claim 12, wherein the RNA-virus-based plasmid is an alphavirus-based plasmid.

14. The microparticles of claim 13, wherein the alphavirus-based plasmid is a Sindbis-virus based plasmid.

15. The microparticles of claim 14, wherein the plasmid is pSINCP.

16. The microparticles of claim 9, wherein the DNA vector construct comprises a eukaryotic promoter 5' of viral cDNA which initiates within a cell the 5' to 3' synthesis of RNA from cDNA, wherein the RNA comprises a vector construct which autonomously amplifies in a cell, the vector construct expressing a heterologous nucleic acid sequence.

17. The microparticles of any one of claims 7-16, wherein the polypeptide-containing antigen is derived from a pathogenic organism or a tumor.

18. The microparticles of claim 17, wherein the pathogenic organism is selected from a virus, a bacterium, a fungus and a parasite.

19. The microparticles of claim 17, wherein the pathogenic organism is selected from HIV, hepatitis B virus, hepatitis C virus, meningitis B, *Haemophilus influenza* type B, pertussis, diphtheria, tetanus, and influenza A virus.

20. The microparticles of any one of claim 7-16, wherein the polypeptide-containing antigen is derived from HIV gp120, HIV gp140, HIV gp160, HIV p24gag or HIV p55gag.

21. The microparticles of any one of claims 1-20, further comprising a polynucleotide-containing molecule, a polypeptide-containing molecule, a polysaccharide-containing molecule, a hormone, an enzyme, or an immunological adjuvant, entrapped within the microparticles.

22. The microparticles of any one of claims 1-20, further comprising a second polynucleotide-containing molecule, a polypeptide-containing molecule, a polysaccharide-containing molecule, a hormone, an enzyme, or an immunological adjuvant, adsorbed to the microparticles.

23. The microparticles of claim 21 or 22, wherein the microparticles further comprise a polypeptide-containing antigen.

24. The microparticles of claim 21 or 22, wherein the microparticles further comprise a polynucleotide-containing molecule.

25. The microparticles of claim 21 or 22, wherein the microparticles further comprise an immunological adjuvant.

26. The microparticles of any one of claims 1-25, wherein the microparticles comprise 0.1 to 10 wt% cationic surfactant.

27. The microparticles of any one of claims 1-26, wherein the cationic surfactant is present during formation of the microparticles, and wherein no cationic surfactant removal step is conducted subsequent to formation of the microparticles.

28. The microparticles of any one of claims 1-27, wherein a first portion of the cationic surfactant is bound to the polymer, wherein a second portion of the cationic surfactant forms a complex with the first polynucleotide-containing molecule, wherein the complex is adsorbed on the surface of the microparticles, and wherein the first surfactant portion and the second surfactant portion comprise the same surfactant or different surfactants.

29. The microparticles of claim 28, wherein the first and second surfactant portions comprise the same surfactant.

30. A method of producing the microparticles of any one of claims 1-30, comprising: (a) forming a w/o/w emulsion comprising the polymer and the cationic surfactant; (b) removing the organic solvent from the emulsion, to form the microparticles; and (c) adsorbing the first polynucleotide-containing molecule to the microparticles.

31. The method of claim 30, wherein the microparticles are not subjected to a cationic surfactant removal step subsequent to microparticle formation.

32. Microparticles made according to the method of claim 30 or claim 31.

33. A microparticle composition comprising the microparticles of any one of claims 1-29 and 32 and a pharmaceutically acceptable excipient.

34. The microparticle composition of claim 33, further comprising an immunological adjuvant.

35. The microparticle composition of claim 34, wherein the immunological adjuvant is selected from CpG oligonucleotides, MF59, dsRNA, E. coli heat-labile toxins, phospholipids compounds, and aluminum salts.

36. The microparticle composition of any one of claims 33-35, wherein the microparticle composition is an injectable composition.

37. A microparticle composition comprising (a) the microparticles of any one of claims 1-29 and 32, and (b) additional microparticles comprising a biodegradable polymer and an immunological adjuvant adsorbed on the surface of the additional microparticles.

38. A microparticle composition comprising (a) the microparticles of any one of claims 1-29 and 32, and (b) additional microparticles comprising a biodegradable polymer and an immunological adjuvant entrapped within the additional microparticles.

## Patentansprüche

1. Mikropartikel, umfassend: (a) ein bioabbaubares Polymer, ausgewählt aus einer Poly(alpha-hydroxysäure), einer Polyhydroxybuttersäure, einem Polycaprolacton, einem Polyorthoester, einem Polyanhydrid und einem Polycyanoacrylat; (b) ein kationisches Tensid; und (c) ein erstes Polynukleotid-enthaltendes Molekül, adsorbiert an der Oberfläche der Mikropartikel, wobei das adsorbierte erste Polynukleotid-enthaltende Molekül zwischen 10 und 30 % des Gesamtgewichts der Mikropartikel ausmacht.

2. Mikropartikel nach Anspruch 1, wobei das kationische Tensid Cetyltrimethylammoniumbromid umfasst.

3. Mikropartikel nach Anspruch 1 oder Anspruch 2, wobei die Mikropartikel einen Durchmesser zwischen 200 nm und 20 µm aufweisen.

4. Mikropartikel nach irgendeinem der Ansprüche 1 bis 3, wobei das Polymer eine Polyhydroxybuttersäure, ein Polycaprolacton, ein Polyorthoester, ein Polyanhydrid oder ein Polycyanoacrylat umfasst.

5. Mikropartikel nach irgendeinem der Ansprüche 1 bis 3, wobei das Polymer eine Poly(α-hydroxysäure) umfasst.

6. Mikropartikel nach Anspruch 5, wobei das Polymer eine Poly(α-hydroxysäure), ausgewählt aus Poly(L-Lactid), Poly(D,L-Lactid) und Poly(Lactid-co-Glycolid), umfasst.

7. Mikropartikel nach irgendeinem der Ansprüche 1 bis 6, wobei das erste Polynukleotid-enthaltende Molekül ein immunologisches Adjuvans ist, ein CpG-Oligonukleotid oder dsRNA umfasst, ein Polypeptid-enthaltendes Antigen kodiert, oder ein Vektorkonstrukt ist, das ein Polypeptid-enthaltendes Antigen kodiert.

8. Mikropartikel nach Anspruch 7, wobei das Vektorkonstrukt ein RNA-Vektorkonstrukt ist.

9. Mikropartikel nach Anspruch 7, wobei das Vektorkonstrukt ein DNA-Vektorkonstrukt ist.

10. Mikropartikel nach Anspruch 9, wobei das DNA-Vektorkonstrukt ein Plasmid ist.

11. Mikropartikel nach Anspruch 10, wobei das Plasmid pCMV ist.

12. Mikropartikel nach Anspruch 9, wobei das DNA-Vektorkonstrukt ein Plasmid auf Basis eines RNA-Virus ist.

13. Mikropartikel nach Anspruch 12, wobei das Plasmid auf Basis von RNA-Virus ein Plasmid auf Basis von Alphavirus ist.

14. Mikropartikel nach Anspruch 13, wobei das Plasmid auf Basis von Alphavirus ein Plasmid auf Basis von Sindbis-Virus ist.

15. Mikropartikel nach Anspruch 14, wobei das Plasmid pSINCP ist.

16. Mikropartikel nach Anspruch 9, wobei das DNA-Vektorkonstrukt einen eukaryotischen Promotor 5' von viraler cDNA umfasst, der innerhalb einer Zelle die 5' zu 3'-Synthese von RNA von cDNA initiiert, wobei die RNA ein Vektorkonstrukt umfasst, das sich autonom in einer Zelle amplifiziert, wobei das Vektorkonstrukt eine heterologe Nukleinsäuresequenz exprimiert.

17. Mikropartikel nach irgendeinem der Ansprüche 7 bis 16, wobei das Polypeptid-enthaltende Antigen von einem Pathogenorganismen oder einem Tumor abgeleitet ist.

18. Mikropartikel nach Anspruch 17, wobei der pathogene Organismus von einem Virus, einem Bakterium, einem Pilz und einem Parasit ausgewählt ist.

19. Mikropartikel nach Anspruch 17, wobei der pathogene Organismus aus HIV, Hepatitis B-Virus, Hepatitis C-Virus, Meningitis B, Haemophilus influenza Typ B, Pertussis, Diphtherie, Tetanus und Influenza A-Virus ausgewählt ist.

20. Mikropartikel nach irgendeinem der Ansprüche 7 bis 16, wobei das Polypeptid-enthaltene Antigen von HIV-gp120, HIV-gp140, HIV-gp160, HIV-p24gag oder HIV-p55gag abgeleitet ist.

21. Mikropartikel nach irgendeinem der Ansprüche 1 bis 20, ferner umfassend ein Polynukleotid-enthaltendes Molekül, ein Polypeptid-enthaltendes Molekül, ein Polysaccharid-enthaltendes Molekül, ein Hormon, ein Enzym oder ein immunologisches Adjuvans, eingeschlossen innerhalb der Mikropartikel.

22. Mikropartikel nach irgendeinem der Ansprüche 1 bis 20, ferner umfassend ein zweites Polynukleotid-enthaltendes Molekül, ein Polypeptid-enthaltendes Molekül, ein Polysaccharid-enthaltendes Molekül, ein Hormon, ein Enzym oder ein immunologisches Adjuvans, adsorbiert an die Mikropartikel.

23. Mikropartikel nach Anspruch 21 oder 22, wobei die Mikropartikel ferner ein Polypeptid-enthaltendes Antigen umfassen.

24. Mikropartikel nach Anspruch 21 oder 22, wobei die Mikropartikel ferner ein Polynukleotid-enthaltendes Molekül umfassen.

25. Mikropartikel nach Anspruch 21 oder 22, wobei die Mikropartikel ferner ein immunologisches Adjuvans umfassen.

26. Mikropartikel nach irgendeinem der Ansprüche 1 bis 25, wobei die Mikropartikel 0,1 bis 10 Gew.% kationisches Tensid umfassen.

27. Mikropartikel nach irgendeinem der Ansprüche 1 bis 26, wobei das kationische Tensid während der Bildung der Mikropartikel vorliegt und wobei kein kationischer Tensid-Entfernungsschritt im Anschluss an die Bildung der Mikropartikel durchgeführt wird.

28. Mikropartikel nach irgendeinem der Ansprüche 1 bis 27, wobei ein erster Abschnitt des kationischen Tensids an das Polymer gebunden ist, wobei ein zweiter Abschnitt des kationischen Tensids einen Komplex mit dem ersten Polynukleotid-enthaltenden Molekül bildet, wobei der Komplex auf die Oberfläche der Mikropartikel adsorbiert ist und wobei der erste Tensidabschnitt und der zweite Tensidabschnitt das gleiche Tensid oder verschiedene Tenside umfassen.

29. Mikropartikel nach Anspruch 28, wobei die ersten und zweiten Tensidabschnitte das gleiche Tensid umfassen.

30. Verfahren zur Herstellung der Mikropartikel nach irgendeinem der Ansprüche 1 bis 30, umfassend: (a) Bilden einer W/O/W-Emulsion, umfassend das Polymer und das kationische Tensid; (b) Entfernen des organischen Lösemittels aus der Emulsion, um die Mikropartikel zu bilden; und (c) Adsorbieren des ersten Polynukleotid-enthaltenden Moleküls an die Mikropartikel.

31. Verfahren nach Anspruch 30, wobei die Mikropartikel im Anschluss an die Mikropartikelbildung keinem kationischen Tensid-Entfernungsschritt unterzogen werden.

32. Mikropartikel, hergestellt gemäß dem Verfahren nach Anspruch 30 oder Anspruch 31.

33. Mikropartikelzusammensetzung, umfassend die Mikropartikel von irgendeinem der Ansprüche 1 bis 29 und 32 und einen pharmazeutisch annehmbaren Exzipienten.

34. Mikropartikelzusammensetzung nach Anspruch 33, ferner umfassend ein immunologisches Adjuvans.

35. Mikropartikelzusammensetzung nach Anspruch 34, wobei das immunologische Adjuvans aus CpG-Oligonukleotiden, MF59, dsRNA, E. coli-hitzelabilen Toxinen, Phospholipidverbindungen und Aluminiumsalzen ausgewählt ist.

36. Mikropartikelzusammensetzung nach irgendeinem der Ansprüche 33 bis 35, wobei die Mikropartikelzusammensetzung eine injizierbare Zusammensetzung ist.

37. Mikropartikelzusammensetzung, umfassend (a) die Mikropartikel von irgendeinem der Ansprüche 1 bis 29 und 32, und (b) zusätzliche Mikropartikel, die ein bioabbaubares Polymer und ein immunologisches Adjuvans, adsorbiert an der Oberfläche der zusätzlichen Mikropartikel, umfassen.

38. Mikropartikelzusammensetzung, umfassend (a) die Mikropartikel nach irgendeinem der Ansprüche 1 bis 29 und 32, und (b) zusätzliche Mikropartikel, umfassend ein bioabbaubares Polymer und ein immunologisches Adjuvans, eingeschlossen innerhalb der zusätzlichen Mikropartikel.

## Revendications

1. Microparticules comprenant : (a) un polymère biodégradable choisi parmi un poly(acide alpha-hydroxylé), un polyacide hydroxybutyrique, une polycaprolactone, un polyorthoester, un polyanhydride et un polycyanoacrylate ; (b) un tensioactif cationique ; et (c) une première molécule contenant un polynucléotide adsorbée sur la surface des microparticules, la première molécule contenant un polynucléotide adsorbé constituant entre 10 et 30 pour cent du poids total des microparticules.

2. Microparticules selon la revendication 1, dans lesquelles le tensioactif cationique comprend du bromure de cétyltriméthylammonium.

3. Microparticules selon la revendication 1 ou la revendication 2, les microparticules ayant un diamètre entre 200 nanomètres et 20 microns.

4. Microparticules selon l'une quelconque des revendications 1 à 3, dans lesquelles le polymère comprend un polyacide hydroxybutyrique, une polycaprolactone, un polyorthoester, un polyanhydride, ou un polycyanoacrylate.

5. Microparticules selon l'une quelconque des revendications 1 à 3, dans lesquelles le polymère comprend un poly(acide α-hydroxylé).

6. Microparticules selon la revendication 5, dans lesquelles le polymère comprend un poly(acide α-hydroxylé), choisi parmi un poly(L-lactide), un poly(D,L-lactide) et un poly(lactide-co-glycolide).

7. Microparticules selon l'une quelconque des revendications 1 à 6, dans lesquelles la première molécule contenant un polynucléotide est un adjuvant immunologique, comprend un oligonucléotide CpG ou un ARNdb, code pour un antigène contenant un polypeptide, ou est une construction de vecteur qui code pour un antigène contenant un polypeptide.

8. Microparticules selon la revendication 7, dans lesquelles la construction de vecteur est une construction de vecteur d'ARN.

9. Microparticules selon la revendication 7, dans lesquelles la construction de vecteur est une construction de vecteur d'ADN.

10. Microparticules selon la revendication 9, dans lesquelles la construction de vecteur d'ADN est un plasmide.

11. Microparticules selon la revendication 10, dans lesquelles le plasmide est le pCMV.

12. Microparticules selon la revendication 9, dans lesquelles la construction de vecteur d'ADN est un plasmide à base de virus à ARN.

13. Microparticules selon la revendication 12, dans lesquelles le plasmide à base de virus à ARN est un plasmide à base d'alphavirus.

14. Microparticules selon la revendication 13, dans lesquelles le plasmide à base d'alphavirus est un plasmide à base de virus Sindbis.

15. Microparticules selon la revendication 14, dans lesquelles le plasmide est le pSINCP.

16. Microparticules selon la revendication 9, dans lesquelles la construction de vecteur d'ADN comprend un promoteur eucaryote en 5' de l'ADNc viral qui initie au sein d'une cellule la synthèse de 5' à 3' de l'ARN à partir de l'ADNc, l'ARN comprenant une construction de vecteur qui s'amplifie de façon autonome dans une cellule, la construction de vecteur exprimant une séquence d'acide nucléique hétérologue.

17. Microparticules selon l'une quelconque des revendications 7 à 16, dans lesquelles l'antigène contenant un polypeptide est dérivé d'un organisme pathogène ou d'une tumeur.

18. Microparticules selon la revendication 17, où l'organisme pathogène est choisi parmi un virus, une bactérie, un champignon et un parasite.

19. Microparticules selon la revendication 17, où l'organisme pathogène est choisi parmi le VIH, le virus de l'hépatite B, le virus de l'hépatite C, la méningite B, *Haemophilus influenza* de type B, la coqueluche, la diphtérie, le tétanos, et le virus de la grippe A.

20. Microparticules selon l'une quelconque des revendications 7 à 16, dans lesquelles l'antigène contenant un polypeptide dérivé de la gpl20 du VIH, la gp140 du VIH, la gp160 du VIH, la p24gag du VIH ou la p55gag du VIH.

21. Microparticules selon l'une quelconque des revendications 1 à 20, comprenant en outre une molécule contenant un polynucléotide, une molécule contenant un polypeptide, une molécule contenant un polysaccharide, une hormone, une enzyme, ou un adjuvant immunologique, incorporé au sein des microparticules.

22. Microparticules selon l'une quelconque des revendications 1 à 20, comprenant en outre une seconde molécule contenant un polynucléotide, une molécule contenant un polypeptide, une molécule contenant un polysaccharide, une hormone, une enzyme, ou un adjuvant immunologique, adsorbé sur les microparticules.

23. Microparticules selon la revendication 21 ou 22, les microparticules comprenant en outre un antigène contenant un polypeptide.

24. Microparticules selon la revendication 21 ou 22, les microparticules comprenant en outre une molécule contenant un polynucléotide.

25. Microparticules selon la revendication 21 ou 22, les microparticules comprenant en outre un adjuvant immunologique.

26. Microparticules selon l'une quelconque des revendications 1 à 25, les microparticules comprenant 0,1 à 10 % en poids de tensioactif cationique.

27. Microparticules selon l'une quelconque des revendications 1 à 26, dans lesquelles le tensioactif cationique est présent durant la formation des microparticules, et aucune étape d'élimination du tensioactif cationique n'étant conduite à la suite de la formation des microparticules.

28. Microparticules selon l'une quelconque des revendications 1 à 27, dans lesquelles une première partie du tensioactif cationique est liée au polymère, dans lesquelles une seconde partie du tensioactif cationique forme un complexe avec la première molécule contenant un polynucléotide, où le complexe est adsorbé sur la surface des microparticules, et où la première partie du tensioactif et la seconde partie du tensioactif comprennent le même tensioactif ou des tensioactifs différents.

29. Microparticules selon la revendication 28, dans lesquelles les première et seconde parties de tensioactif comprennent le même tensioactif.

30. Procédé de production des microparticules selon l'une quelconque des revendications 1 à 30, comprenant : (a) la formation d'une émulsion e/h/e comprenant le polymère et le tensioactif cationique ; (b) l'élimination du solvant organique à partir de l'émulsion, pour former les microparticules ; et (c) l'adsorption de la première molécule contenant un polynucléotide sur les microparticules.

31. Procédé selon la revendication 30, dans lequel les microparticules ne sont pas soumises à une étape d'élimination du tensioactif cationique à la suite de la formation des microparticules.

32. Microparticules fabriquées selon le procédé de la revendication 30 ou de la revendication 31.

33. Composition de microparticules comprenant les microparticules selon l'une quelconque des revendications 1 à 29 et 32 un excipient pharmaceutiquement acceptable.

34. Composition de microparticules selon la revendication 33, comprenant en outre un adjuvant immunologique.

35. Composition de microparticules selon la revendication 34, dans laquelle l'adjuvant immunologique est choisi parmi des oligonucléotides CpG, MF59, de l'ARNdb, des toxines thermolabiles d'E. *coli,* des composés phospholipides, et des sels d'aluminium.

36. Composition de microparticules selon l'une quelconque des revendications 33 à 35, la composition de microparticules étant une composition injectable.

37. Composition de microparticules comprenant (a) les microparticules selon l'une quelconque des revendications 1 à 29 et 32, et (b) des microparticules supplémentaires comprenant un polymère biodégradable et un adjuvant immunologique adsorbé sur la surface des microparticules supplémentaires.

38. Composition de microparticules comprenant (a) les microparticules selon l'une quelconque des revendications 1 à 29 et 32, et (b) des microparticules supplémentaires comprenant un polymère biodégradable et un adjuvant immunologique incorporé au sein des microparticules supplémentaires.
